# EUROPEAN PATENT SPECIFICATION

(11) **EP 4 017 560 B1**
(45) Date of publication and mention of the grant of the patent: **23.09.2026**
(21) Application number: 20764254.7
(22) Date of filing: 17.08.2020
(51) Int. Cl.: A61M 5/32, A61M 5/142, A61M 5/20

(54) **DRUG DELIVERY DEVICE WITH CONFIGURABLE NEEDLE SHIELD ENGAGEMENT COMPONENTS AND RELATED METHODS**
ARZNEIMITTELABGABEVORRICHTUNG MIT KONFIGURIERBAREN NADELSCHUTZEINGRIFFSKOMPONENTEN UND ZUGEHÖRIGE VERFAHREN
DISPOSITIF D'ADMINISTRATION DE MÉDICAMENT DOTÉ DE COMPOSANTS CONFIGURABLES DE MISE EN PRISE DE PROTECTION D'AIGUILLE ET MÉTHODES ASSOCIÉES

(30) Priority: 23.08.2019 US 201962891042 P
(43) Date of publication of application: 29.06.2022
(73) Proprietor: Amgen Inc., Thousand Oaks, CA 91320-1799 (US)
(72) Inventor: DASOJU, Sunitha, Thousand Oaks, CA 91320-1799 (US); RAHBARI, Azita, Thousand Oaks, CA 91320-1799 (US); RICH, William, Thousand Oaks, CA 91320-1799 (US); ANTONINI, Antonio, Thousand Oaks, CA 91320-1799 (US); ALAGIA, Nicola, Antonio, Thousand Oaks, CA 91320-1799 (US); TOSARINI, Angelo, Thousand Oaks, CA 91320-1799 (US)
(74) Representative: J A Kemp LLP
(86) International application number: PCT/US2020/046598
(87) International publication number: WO 2021/041067

(56) References cited:
- WO-A1-2012/103140
- WO-A1-2013/058697
- WO-A1-2018/069031

## Description

### Field of the Disclosure

The present disclosure generally relates to drug delivery devices and, more particularly, to drug delivery devices having configurable needle shield engagement components.

### Background

Drugs can be administered through the use of drug delivery devices such as autoinjectors or on-body injectors. Autoinjectors and on-body injectors may be used to help automate the injection and delivery or administration process, thereby simplifying the process for certain patient groups or sub-groups for which use of the syringe/vial combination or pre-filled syringe systems would be disadvantageous, whether because of physiological or psychological barriers, form factors, or ergonomic considerations. WO 2012/103140, WO 2013/058697 and WO 2018/069031 disclose various arrangements for removing needle shields.

Due to aversions to exposed needles, as well as health and safety issues that may be involved, various injectors and shields have been developed for concealing needles from the user. In one example, a syringe for an injector may be provided with a needle shield that is engaged by a cap. With this configuration, when use is desired, a user can grip and pull the cap to thereby remove the needle shield from the syringe so that the injector is ready to deliver an injection Current injectors can be configured to receive a variety of sizes and types of syringes therein, however, which can be equipped with both needle shields having non-rigid outer walls and needle shields having rigid outer walls. Accordingly, a universal cap is needed that is configured for use with an injector while accommodating needle shields with non-rigid outer walls and rigid outer walls.

### Summary

The invention is defined in the independent claims. Further aspects and preferred embodiments are defined in the dependent claims. Any aspects, embodiments and examples of the present disclosure which do not fall under the scope of the appended claims do not form part of the invention and are merely provided for illustrative purposes. In accordance with a first aspect, a drug delivery device is disclosed that includes a housing, an injection assembly, a cassette, a syringe disposed within the cassette and comprising a reservoir containing a drug and a needle fluidly coupled to the reservoir, and a cap assembly kit. The cap assembly kit includes a cap housing having an internal cavity sized to interchangeably receive one of a first remover or a second remover. The first remover has a sidewall defining a central passage and is configured to receive a first type of needle shield and the second remover has a sidewall defining a central passage and is configured to receive a second type of needle shield.

According to some forms, the drug delivery device can include one or more of the following aspects: the first type of needle shield can have a non-rigid outer wall and the second type of needle shield can have a rigid outer wall; the first remover and the second remover can each be configured to be insertably mounted within the internal cavity of the cap housing; or the cap can include a plurality of arms that extend along the longitudinal axis thereof, where the arms are each resiliently flexible and have an inwardly extending retention surface to engage the remover as the remover is inserted into the internal cavity of the cap housing past the retention surface.

The first remover includes a plurality of retention portions that extend inwardly into the central passage of the first remover. In some examples, the plurality of retention portions can be angled to extend distally within the cap housing and have distally oriented pointed ends or the plurality of retention portions can be angled to extend distally within the cap housing and have flat distal ends. In yet further examples, the plurality of retention portions can extend from a distal edge of the sidewall.

The second remover includes a plurality of retention portions that extend inwardly into the central passage of the second remover. In some examples, the drug delivery device can include one or more of the following aspects: the plurality of retention portions of the first remover and the plurality of retention portions of the second remover can differ from each other, the plurality of retention portions can include engagement edges that extend generally parallel to a longitudinal axis of the second remover, the engagement edges can have a blunted configuration, the plurality of retention portions can be disposed in a proximal axial position of the second remover, or the sidewall can include a crenellation configuration with the plurality of retention portions extending radially inward from longitudinal edges of portions of the crenellation configuration.

In accordance with a second aspect, a method of assembly for a drug delivery device cassette is disclosed that includes providing a cap assembly that includes a cap housing having an internal cavity with a rearward opening, selecting one of a first remover configured to receive a first type of needle shield and a second remover configured to receive a second type of needle shield, orienting the selected one of the first remover and second remover to be coincident with a longitudinal axis of the cap housing, and inserting the selected one of the first remover and second remover into the internal cavity of the cap housing.

According to some forms, selecting the one of the first remover and the second remover can include selecting the one of the first remover and the second remover based on whether a needle shield for the drug delivery device cassette has a rigid or non-rigid outer wall. Further, in some versions, the first and second removers can each have a different plurality of retention portions that extend radially inwardly from an annular sidewall thereof, and selecting the one of the first remover and the second remover can include selecting the one of the first remover and the second remover based on whether the plurality of retention portions thereof are configured to grip a needle shield having a rigid or a non-rigid outer wall.

According to further forms, the method can include providing a cassette, a syringe disposed within the cassette and comprising a reservoir containing a drug and a needle fluidly coupled to the reservoir, and a needle shield disposed over the needle, and coupling the cap assembly to the cassette so that the needle shield is disposed within the internal cavity of the cap housing and through the selected one of the first remover and second remover with the plurality of retention portions gripping an outer wall of the needle shield. If desired, the method can also optionally include restricting linear and rotational motion of the needle shield with the remover and/or extracting the cap assembly from the cassette to thereby uncouple the needle shield from the syringe and the needle.

In accordance with a third aspect, a cap assembly for a drug delivery device is described that includes a cap housing defining an internal cavity with a rearward opening at a proximal end to receive a needle shield along a longitudinal axis and a remover insertably mounted within the internal cavity of the cap housing and having a sidewall with a generally annular configuration. A plurality of retention portions of the remover extend radially inwardly from the sidewall within the internal cavity, such that the plurality of retention portions are configured to grip a needle shield inserted through the remover within the internal cavity of the cap housing.

According to some forms, the plurality of retention portions can be angled to extend distally within the cap housing. The retention portions can also each include a distally oriented point and/or can extend from a distal edge of the sidewall. Additionally, the cap assembly can be provided in combination with a cassette, a syringe disposed within the cassette and including a reservoir containing a drug and a needle fluidly coupled to the reservoir, and a needle shield having a non-rigid outer wall disposed over the needle, where the cap assembly is coupled to the cassette with the needle shield disposed within the internal cavity of the cap housing and through the remover with the plurality of retention portions gripping the non-rigid outer wall such that extraction of the cap assembly from the cassette uncouples the needle shield from the needle.

According to some forms, the sidewall can include a crenellation configuration at an end thereof with the plurality of retention portions extending radially inward from longitudinal edges of portions of the crenellation configuration. If desired, the plurality of retention portions can extend inwardly away from the sidewall transverse to the longitudinal axis with internal engagement edges of the plurality of retention portions extending generally parallel to longitudinal axis. Additionally, the cap assembly can be provided in combination with a cassette, a syringe disposed within the cassette and including a reservoir containing a drug and a needle fluidly coupled to the reservoir, and a needle shield having a rigid outer wall disposed over the needle, where the cap assembly is coupled to the cassette with the needle shield disposed within the internal cavity of the cap housing and through the remover with the plurality of retention portions gripping the rigid outer wall such that extraction of the cap assembly from the cassette uncouples the needle shield from the needle.

According to any of the above forms, the cap housing can define opposing retention surfaces within the internal cavity spaced along the longitudinal axis thereof, where the retention surfaces are spaced apart a distance to removably receive the remover therebetween. In further forms, the cap can include a plurality of arms that extend along the longitudinal axis, where the arms have an inwardly extending retention tab to engage the remover as the remover is inserted into the internal cavity of the cap housing. If desired, the plurality of arms can be configured to resiliently flex outwardly to allow the remover to be inserted and removed from the internal cavity of the cap housing.

According to any of the above forms, the cap housing and the remover can be composed of different materials and/or the plurality of retention portions can be disposed in an array of retention portions of the sidewall disposed symmetrically around the remover.

In accordance with a second aspect, a cap assembly kit for a drug delivery device cassette assembly including a cassette, a syringe disposed within the cassette and comprising a reservoir containing a drug and a needle fluidly coupled to the reservoir, and a needle shield disposed over the needle is described. The cap kit assembly includes a cap housing having an internal cavity sized to receive the needle shield at least partially therein, a first remover configured to be removably mounted within the internal cavity of the cap housing, where the first remover has a sidewall defining a central passage and a plurality of retention portions extending inwardly into the central passage, and a second remover configured to be removably mounted within the internal cavity of the cap housing, the second remover having a sidewall defining a central passage and a plurality of retention portions extending inwardly into the central passage. Further, the plurality of retention portions of the first remover and the plurality of retention portions of the second remover are different.

In accordance with a fourth aspect, a method of assembly for a drug delivery device cassette is described that includes providing a cap assembly including a cap housing having an internal cavity with a rearward opening and a remover having a sidewall with a generally annular configuration, orienting the remover to be coincident with a longitudinal axis of the cap housing, and inserting the remover into the internal cavity of the cap housing so that a plurality of retention portions of the remover extend radially inwardly from the sidewall within the internal cavity.

### Brief Description of the Drawings

The above needs are at least partially met through provision of the embodiments described in the following detailed description, particularly when studied in conjunction with the drawings, wherein:
Figure 1 is a diagrammatic view of an example autoinjector drug delivery device in accordance with various embodiments;
Figure 2 is a side view of an example autoinjector apparatus that includes a cassette and an autoinjector showing a cassette prior to installation in the autoinjector;
Figure 3 is an end view of an end of the autoinjector of the autoinjector apparatus of Figure 1;
Figure 4 is an exploded perspective view of an example cassette for the autoinjector apparatus of Figure 1;
Figure 5A is a sectional side view of a first example drug container that can be provided in the cassette of Figure 4;
Figure 5B is a side view of a second example drug container that can be provided in the cassette of Figure 4;
Figure 6A is a top down front perspective view of the cassette of Figure 4;
Figure 6B is a sectional side view of the cassette of Figure 6A;
Figure 7A is a perspective rear view of an example cassette cap for the cassette of Figure 4;
Figure 7B is a sectional side view of the proximal end of a cassette showing the cassette cap of Figure 7A coupled to a needle shield of a drug container provided in the cassette;
Figure 7C is a bottom up front perspective view of a portion of the cassette with the cassette cap of Figure 7A removed from the cassette.
Figure 7D is a sectional side view of the proximal portion of the cassette installed in the autoinjector showing the operation of a cantilever lock arm of the cassette cap of Figure 7A;
Figure 8A is a top down front perspective view of a proximal portion of the outer housing of the cassette of Figure 4 with the cassette cap of Fig. 7A removed, showing an embodiment of a slot for receiving a key portion of the cassette cap;
Figure 8B is a top down front perspective view of the cassette of Figure 4 showing how an anti-rotation structure formed by the slot of the outer housing and the key of the cassette cap prevents the cassette cap of Figure 7A from being rotated or twisted around its longitudinal axis Z when the cassette cap is in the cassette (prior to needle shield removal) and thus, prevents rotation of the needle shield;
Figure 9 is an exploded perspective view of a first example cap assembly including a cassette cap and a remover;
Figure 10 is a cross-sectional view of the cap assembly of Figure 9;
Figure 11 is an exploded perspective view of a second example cap assembly including a cassette cap and a remover;
Figure 12 is an exploded perspective view of a third example cap assembly including a cassette cap and a remover; and
Figure 13 is a diagrammatic view of an on-body injector drug delivery device in accordance with various embodiment.

### DETAILED DESCRIPTION

A cap assembly is disclosed herein that is configured to engage and retain a needle shield, such that extraction of the cap assembly from a device also uncouples the needle shield from an injection needle. The cap assembly includes a cap housing and a remover that can be inserted into the cap housing. The single cap housing with selectable removers allows reusable drug delivery devices, such as autoinjectors and on-body injectors, to accommodate syringes or other drug containers that have both rigid and non-rigid needle shields. In other words, a remover can be selected that will engage and retain a particular needle shield, whether the needle shield has a rigid or non-rigid outer wall. The remover, in combination with the cap housing, can also be configured to hold the needle shield to restrict linear and rotational motion thereof.

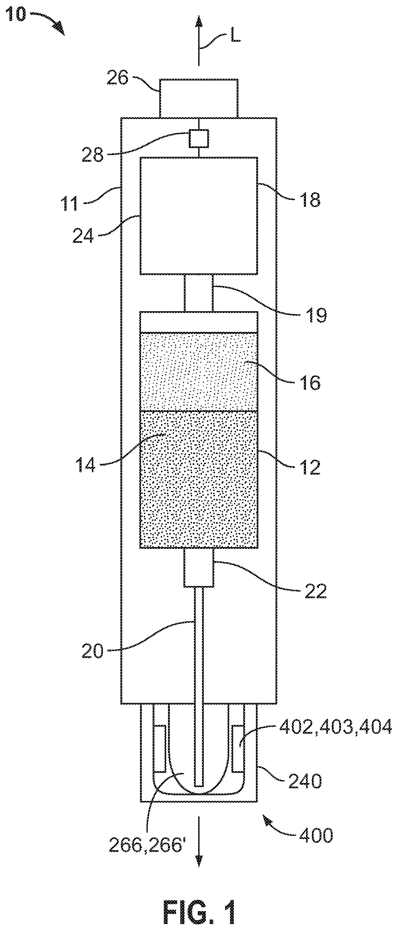

In some versions as illustrated in Fig. 1, drug delivery devices 10, such as autoinjectors, can have a vertically oriented configuration with some or all drug delivery components, including an injection assembly, disposed in stacked relation along a longitudinal axis L within a housing 11 of the devices 10. As a more specific example, the devices 10 can be configured to operate and inject a user with the device 10 oriented generally perpendicular to a skin surface of the user. The drug delivery components can include a reservoir 12 having a drug 14 contained therein, a stopper 16 disposed within the reservoir 12 and sildably movable therein along the longitudinal axis L, a needle 20 oriented along the longitudinal axis L, and a flow path 22 fluidly coupling the reservoir 12 to the needle 20. The components can further include an injection assembly that includes a drive mechanism 18 coupled to a plunger 19 to drive the stopper 16 through the reservoir 12 and a needle insertion mechanism 24 configured to insert the needle 20 to a desired subcutaneous depth within the user. By some approaches, the needle insertion mechanism 24 can be a retractable needle guard to expose the needle 20 or a drive mechanism to longitudinally move the needle a desired distance. For example, the drive mechanism 18 can be configured to drive both movement of the stopper 16 and the needle 20 by moving some or all of the reservoir 12, flow path 22, and needle 20. As commonly configured, one or more of the components of the device 10, such as the drive mechanism 18 and needle insertion mechanism 24, can be operable in response to actuation of a user input device 26 accessible on an exterior of the housing 11. Suitable drive mechanisms include, but are not limited to, springs, gas sources, phase changing materials, motors, or other electromechanical systems. Pursuant to this, the device 10 can include electronic components, such as a controller 28, to control operation of one or more of the drug delivery components. It will be understood that although Fig. 1 shows the components centered along the longitudinal axis L, one or more of the components can be disposed off center from the longitudinal axis L within the housing 11 and still be considered to be in a stacked relation. In one example, an autoinjector drug delivery device having drug delivery components in a stacked relation corresponds to the reservoir 12 co-axially aligned with the needle 20. As described in more detail below, the device includes a cap assembly 400 that includes a cap housing 240 and a remover 402, 403, 404. The device 10 can further include a needle shield 266, 266' disposed over the needle 20 in a storage state, where the needle shield 266, 266' is engaged and retained by the remover 402, 403, 404. The needle shield 266, 266' is removable by extraction of the cap assembly 400 from the device 10. Example autoinjector devices are described in US Serial No. 62/447,174, filed January 17, 2017.

Fig. 2 shows an embodiment of an autoinjector system or apparatus 100 that can be used for injecting a dose of pharmaceutical product (drug) into a patient, the injection often being self-administered by the patient (user). Alternatively, the drug can be administered by a health-care provider. As shown, the autoinjector system or apparatus 100 may comprise a removable cassette 200 and an autoinjector 300. Various embodiments of the cassette 200 may be constructed to contain a drug to be injected into the user by the autoinjector 300. In various other embodiments the cassette 200 may be constructed for use in training the user to operate the autoinjector 300 (a training cassette). The autoinjector 300 may be constructed to deliver an injection automatically upon actuation by the user or some other person. Various embodiments of the autoinjector 300 may have a cassette door 308 that can be constructed to pivot between and an open position and a closed position to allow insertion of the cassette 200 into the autoinjector 300. In some embodiments, the cassette door 308 may include a "cassette" icon (not shown) that indicates the insertion entry point for the cassette 200.

Referring to Fig. 3, various embodiments of the autoinjector 300 may comprise a casing 302 having a handle section 304 and a cassette receiving section 306 in line with the handle section 304. The cassette receiving section 306 comprises a cassette door 308. The cassette door 308 receives the cassette 200 in an open position (Fig. 2) and aligns the cassette 200 with insertion and extrusion drives, and other structures and components of the autoinjector 300 in a closed position. The cassette receiving section 306 of the casing 302 and the cassette door 308 may form a proximal end wall 318 of the autoinjector 300. The proximal end wall 318 may be configured as a broad, flat and stable base for easily positioning the autoinjector 300 on a support surface, after removal of a shield remover 240 (Fig. 6A), described in more detail below, or when the autoinjector 300 does not contain the cassette 200. The portion of the proximal end wall 318 formed by the cassette door 308 may include an aperture 308A that is sized and shaped to allow the shield remover 240 to be removed from the cassette 200 and withdrawn through the aperture 308A, when the cassette 200 is installed in the autoinjector 300. The proximal end wall of the autoinjector 300 may further comprise a target light 320. The target light 320 may be constructed to turn on when the shield remover 240 is removed from the cassette 200 and withdrawn through the aperture 308A, thereby visually indicating that the shield remover 240 has been removed. Once turned on, the target light aids the user in visualizing and selecting an injection site.

Referring now to Fig. 4, various embodiments of the cassette 200 may comprise an outer housing 210, an inner sleeve 220, a drug container 260 for containing a drug, a cassette cap 240, a lock cap 230, and a cover 250. Such embodiments of the cassette 200 facilitate and enable easy injection of the drug with the autoinjector and can be constructed for a single, disposable use. In various embodiments, the lock cap 230 and cover 250 of the cassette 200 may be constructed to resist removal of the drug container 260 from the cassette 200, thereby preventing needle sticks before and after use of the cassette 200 and also preventing the drug container 260 from being taken out of the cassette 200 or replaced. In addition, the lock cap 230 and cover 250 protect the drug container 260 during shipment and transportation. The cassette cap 240, in various embodiments, may be constructed to remove a needle shield 266 covering an injection needle associated with the drug container 260. In various other embodiments, the cassette cap 240 may also be constructed to engage the outer housing 210 of the cassette 200, such that the cassette cap 240 cannot be rotated or twisted, thereby preventing the needle shield 266 from damaging the injection needle. Various embodiments of the inner sleeve 220 may be constructed to position the drug container 260 within the cassette housing 210 in either a needle-concealed position or a needle injection position during an injection cycle of the autoinjector. In various other embodiments, the outer housing 210 and the inner sleeve 220 of the cassette 200 may include one or more locking arrangements that protect the drug container 260 and prevent unintended needle exposure or damage. Various other embodiments of the cassette 200 may include a cassette identification arrangement that interfaces with the autoinjector to communicate the installation of the cassette 200 within the autoinjector and/or information about the cassette 200.

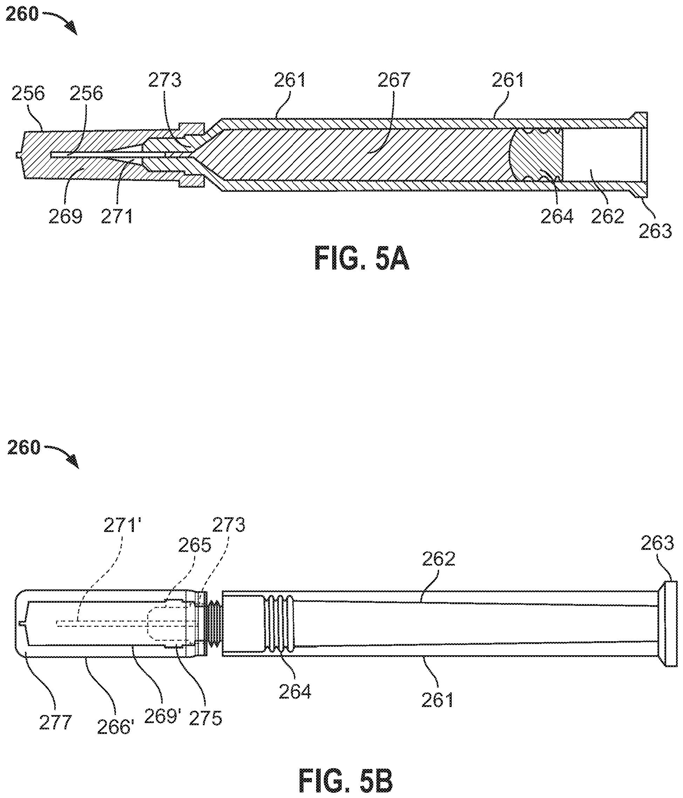

As shown in Figs. 5A and 5B, the drug container 260 may comprise a conventional glass or plastic syringe comprising a barrel 261 that defines a fluid chamber 262. The fluid chamber 262 may be filled for treatment or be prefilled with a predetermined dose of a drug 267. The drug may have a viscosity that depends on the temperature of the product. The syringe 260 may further comprise an injection needle 265 removably or fixedly disposed at a proximal end of the barrel 261, and an outwardly extending flange 263 disposed at a distal end of the barrel 261. The injection needle 265 may communicate with the fluid chamber 262 to allow dispensing of the predetermined dose of the drug 267 expelled from the fluid chamber 262 of the syringe barrel 261. The syringe 260 may further comprise a moveable plunger-stopper 264, disposed within the fluid chamber 262 of the barrel 260, for expelling the predetermined dose of the drug 267 from the chamber 261 so that it may be dispensed through the injection needle 265. In a first form, shown in Fig. 5A, a protective needle shield 266 made of a non-rigid material may be provided for covering the injection needle 265. As shown, the needle shield 266 of this form is a unitary component with a sidewall 269 defining a cavity 271 to receive the injection needle 265 and at least a portion of a needle hub 273 of the syringe 260 therein. In a second form, shown in Fig. 5B, a protective needle shield 266' that includes a rigid material or component may be provided for covering the injection needle 265. As shown, the protective needle shield 266' of this form includes a non-rigid core 275 configured similarly to the needle shield 266 shown in Fig. 5A with a sidewall 269' defining a cavity 271' to receive the injection needle 265 and at least a portion of the needle hub 273 therein. Further, the protective needle shield 266' includes a rigid outer shell or casing 277 with a sidewall that extends around the non-rigid core 275. As shown, the shell or casing 277 may have an asymmetrical configuration, which can be a result of openings therethrough.

Referring collectively to Figs. 6A-6B, various embodiments of the outer housing 210 of the cassette 200 may comprise a top wall 210t, a bottom wall 210b, side walls 210s connecting the top and bottom walls 210t and 210b, respectively, a front or proximal end wall 210pe and an open rear or distal end 210de. The proximal end wall 210pe of the outer housing 210 may include an aperture 214 which is constructed to removably receive the cassette cap 240. The outer housing 210 may be constructed to retain the inner sleeve 220 therein while allowing it to be freely moved within the outer housing 210 in a slidable manner after removal of the cassette cap 240. Some embodiments of the outer housing 210 may comprise an elongated opening or window 212 in each side wall 210s thereof. The outer housing 210 of the cassette 200 may also include a pin 215 (Fig. 6A) or any other suitable mechanical structure that prevents the cassette 200 from being inserted into the cassette door in the wrong direction and/or orientation. An "arrow" icon may be provided on the outer housing 210 (not shown) to indicate the proper direction and orientation for inserting the cassette into the cassette door.

Referring still to Figs. 6A-6B, various embodiments of the inner sleeve 220 may comprise proximal and distal ends 222 and 224, respectively. The sleeve 220 may be sized and dimensioned to directly or indirectly hold the drug container 260 therein in a secure manner. The proximal end 222 of the inner sleeve 220 may define an aperture 222a which is constructed to allow the injection needle 265 of the drug container 260 to extend therethrough. The inner sleeve 220 may further comprise a drive post 268, which allows it to be driven by the insertion drive of the autoinjector during the needle insertion cycle of the autoinjector's injection cycle. The inner sleeve 220 can be driven through the outer housing 210 of the cassette 200 by the insertion drive of the autoinjector, during which the drug container 260 moves from a distal position in the outer housing 210 to a proximal position in the outer housing 210 and then back to the distal position. When the inner sleeve 220 is in the distal position (needle-concealed position), the injection needle of the drug container 260 is contained within the outer housing 210 of the cassette 200 and concealed from view by the user. When the inner sleeve 220 is in the proximal position (needle-injection position), the injection needle of the drug container 260 extends out through the aperture 214 in the proximal end wall 210pe the outer housing 210 of the cassette 200 and the autoinjector (not shown). The lock cap 230 closes the open distal end 224 of the inner sleeve 220 thereby locking the drug container 260 within the inner sleeve 220, so that the drug container 260 moves with the inner sleeve 220 as it is driven forward or backward through the outer housing 210 by the insertion drive of the autoinjector, during the insertion cycle of the autoinjector 300. The cover 250 closes the open distal end 210de of the outer housing 210 and prevents tampering with the drug container 260 by encasing the inner sleeve 220 and the drug container 260 within the outer housing 210 of the cassette 200, and also completes the cosmetic appearance of the cassette 200. The inner sleeve 220 may be made from a transparent, rigid material, such as a clear polycarbonate, to allow viewing of the drug container 260 through the windows 212 in the side walls 210s of the outer housing 210.

Figs. 7A and 7B collectively show one example embodiment of the cassette cap 240 of the cassette 200. The cassette cap 240 may function as a needle shield remover by engaging and gripping the needle shield 266 of the drug container 260 in a manner that allows the user to remove the needle shield 266 from the drug container 260, prior to operating the autoinjector 300. Further, the cassette cap 240 may lockingly engage the cassette outer housing 210 so that it cannot be easily withdrawn from the cassette 200 unless the cassette 200 is properly installed in the autoinjector. This prevents the needle shield 266 from being inadvertently removed from the drug container 260 when, for example, the cassette 200 is handled by the user. In addition, the presence of the shield remover 240 provides an indication that the cassette 200 has not been previously used or tampered with.

As shown in Fig. 7A, various embodiments of the cassette cap 240 may comprise a hollow body 241 formed by a generally cylindrical portion 241c and a generally rectangular, key portion (key) 241k disposed lateral to and merging with the cylindrical portion 241c. The cassette cap 240 may further comprise a tapered portion 242 that extends proximally from the cylindrical portion 241c of the body 241. An outwardly extending flange 244 terminates the tapered portion 242 and closes the cassette cap 240 at a proximal end 240pe thereof. The flange 244 may function as a finger gripping member that allows a user to grip and pull the cassette cap 240 out of the cassette 200 to remove the needle shield 266 from the drug container 260 after the cassette has been properly installed in the autoinjector. To facilitate gripping and pulling of the cassette cap 240, the flange 244 may have a generally oblong shape which is easily gripped by users with dexterity problems. An "arrow" icon 243 may be provided on the tapered portion 242 of the cassette cap 240 to indicate the proper direction and orientation for inserting the cassette into the cassette door of the autoinjector.

The cylindrical portion 241c and the key 241k are open at a distal end 240de of the cassette cap 240. The open distal end of the cylindrical portion 241c may be formed by a plurality of flexible, outwardly flared tongues 245t that define an expandable collar structure 245, which merges with the open distal end of the key 241k. The expandable collar structure 245 prevents the cassette cap 240 from being reinserted into the cassette as shown in Fig. 7C. The cylindrical portion 241c may include flexible members 241cf that allow the cylindrical portion 241c to accept a metal insert 246 (Fig. 7B) that help engage and grip needle shield.

Referring again to Fig. 7A, the key 241k may include an end wall 241kc that closes the proximal end thereof. The end wall 241kb may extend slightly beyond a bottom wall 241kb of the key 241k, thereby forming a stop 241ks.

As shown in Fig. 8A, the proximal end wall 210pe of the cassette outer housing 210 may include a slot 214s that extends from the aperture 214 toward the bottom wall 210b of the housing 210. The slot 214s may be sized and shaped so that it mates with the key 241k of the cassette cap 240 with the leading edge 2101e of the outer housing bottom wall 210b engaging the stop 241ks of the cassette cap key 241k, when the cassette cap 240 is in the cassette 200, thereby forming a cassette cap anti-rotation structure . As shown in Fig. 8B, the anti-rotation structure formed by the slot 214s and key 241k prevents the cassette cap 240 from being rotated or twisted around its longitudinal axis Z when the cassette cap 240 is in the cassette 200 (prior to needle shield removal) and thus, prevents rotation of the needle shield. This is important because rotation of the needle shield can result in cutting or coring of the needle shield by the sharp end of the injection needle. Accordingly, the anti-rotation structure protects the needle shield from being damaged by the injection needle when the cassette cap 240 is in the cassette 200. The stop 241ks of the cassette cap key 241k can limit cassette cap 240 from being pushed along the longitudinal axis Z distal towards the syringe, which also prevents the injection needle from penetrating and thereby damaging the needle shield.

Referring again to Figs. 7A-7C, the bottom wall 241kb of the key 241k may define a cassette cap locking structure formed by a distally extending cantilever spring member 247 and a downwardly extending projection or lock tab 248 provided at the free end of the spring member 247. The lock tab 248 may comprise an undercut formed by an inclined surface 248s that defines an acute angle 0 with the bottom surface 247b of the spring member 247.

As shown in Figs. 7B and 7C, a metal tubular insert 246 may be provided on an interior surface 241i of the cylindrical body portion 241c for gripping the outer surface of the needle shield 266 so that it can be withdrawn with the cassette cap 240. In various other embodiments, the metal tubular insert 246 may be replaced by gripping teeth (not shown) formed on the interior surface 241i of the cylindrical body portion 241c. The cassette cap 240 may extend through the aperture 214 formed in the proximal end wall 210pe of the outer housing 210 of the cassette 200, which locates the flange or gripping member 244 of the cassette cap 240 outside of the cassette 200. The locking structure of the cassette cap 240, formed by the cantilever spring member 247 and lock tab 248, may be disposed within the marginal proximal portion of the outer cassette housing 210, such that it locks the cassette cap 240 in place in the cassette 200, in a tamper-resistant manner. Locking may be facilitated by the cantilever spring member 247, which forces or biases the tab 248 into a lock aperture 210a (Fig. 7C) that may be defined in the bottom wall 210b of the outer housing 210 of the cassette 200. The lock tab 248 engaged with the lock aperture 210a of the cassette outer housing 210, substantially prevents withdrawal of the cassette cap 240 from the cassette 200, unless the cassette 200 is properly installed within the autoinjector. Because the cassette cap 240 is attached to the needle shield 266 and locked within the cassette 200, the needle shield 266 may not be inadvertently removed from the syringe 260, prior to proper installation in the autoinjector. The presence of the cassette cap 240 also provides an indication that the cassette 200 has not been previously used or tampered with.

As shown in Fig. 7C, once the cassette cap 240 has been removed, the tongues 245t of the expandable partial collar structure 245 expand or spread outwardly to prevent the cassette cap 240 and the needle shield 266 attached thereto (not visible) from being re-inserted into the aperture 214 in the proximal end wall 210pe of the cassette outer housing 210. The absence of the cassette cap 240, therefore, provides an indication to the user that the cassette 200 has already been used or has been tampered with.

Fig. 7D shows the cassette 200 after the access door of the autoinjector (both not visible) has been closed. As shown, the cassette 200 is mounted on the support surface 301s of the autoinjector chassis 301. The chassis 301 may include a pin switch P, which is coupled to the microprocessor of the autoinjector in a manner that allows signals or data to be communicated to the microprocessor. Closure of the autoinjector cassette door may cause the pin switch P to press on the lock tab 248 (if certain conditions regarding the cassette are met as will be explained further on), thereby bending the cantilever spring member 247 up, and releasing it from the lock tab 248 from the lock tab receiving aperture 210a (Fig. 7C) in the bottom wall 210B of the outer cassette housing 210, thereby unlocking the cassette cap 240 from the cassette 200. With the locking tab 248 unlocked, a user can now grasp the gripping member 244 of the cassette cap 240 and withdraw it from the cassette 200 and the autoinjector, thereby removing the needle shield 266 and uncovering the injection needle 265. When the pin switch P engages the lock tab 248, it may also signal the autoinjector's microprocessor so that the autoinjector knows that the cassette 200 has been installed.

Details of one example cap assembly 400 suitable for use with a cassette for a drug delivery device, such as an autoinjector or on-body injector device, are shown in Figs. 9-12. The cap assembly 400 includes a cassette cap 240 and one or more removers 402, 403, 404. The cap 240 can be configured as described in the above forms and, as such, only differences will be described with reference to Figs. 9-12. As shown, the flexible members 241cf can be opposing arms formed in the cylindrical portion 241c by cut out portions extending therethrough. A proximal end 406 of each of the flexible members 241cf can include a proximal tab 408 that extends radially inwardly into a cavity 410 formed by the cylindrical body portion 241c. Further, the cylindrical body portion 241c can include one or more distal tabs 412 that are spaced from the proximal tabs 408 along the body portion 241c and that extend into the cavity 410. So configured, the proximal and distal tabs 408, 412 define retention surfaces 414, 416 that face one another within the cavity 410 and are spaced from one another a distance sufficient to receive one of the removers 402, 403, 404 therebetween. Due to the proximal tab 408 being coupled to or integral with the flexible members 241cf, a physician or other person assembling the cap assembly 400 can insert one of the removers 402, 403, 404 into the cavity 410 and push the remover 402, 403, 404 past the proximal tabs 408, which flexes the flexible members 241cf outwardly. After the remover 402, 403, 404 clears the proximal tabs 408, the flexible members 241cf resiliently return radially inwardly to position the proximal tabs 408 within an exit path of the remover 402, 403, 404 thereby preventing the remover 402, 404 from longitudinal movement out of the cap 240. Similarly, if a physician or other person assembling the cap assembly 400 desired to take the remover 402, 403, 404 out of the cap 240, such as to change out to a different remover 402, 403, 404, the user can force the flexible members 241cf outwardly to clear the longitudinal path for the remover 402, 403, 404 to be pulled through the rear opening 240o of the cap 240. The distal tab 412 can be substantially fixed within a longitudinal path of the remover 402, 403, 404 to restrict a depth that the remover 402, 403, 404 can be inserted into the cap 240. In some versions, the tabs 408, 412 can be spaced from one another to provide a small clearance, e.g., 1-3 mm, to receive the remover 402, 403, 404 therebetween.

Example removers 402, 403, 404 are shown in Figs. 9-12. Each of the removers 402, 403, 404 shown in Figures 9-12 has a tubular configuration with an annular sidewall 418, but other configurations are suitable. The sidewall 418 can be continuous or dis-continuous as desired. For example, the sidewall 418 can have a slot opening extending longitudinally therealong, such as when a strip of material is formed into a ring or other shape. The annular sidewall 418 of the illustrated forms defines a cylindrical longitudinal passage 420 therethrough. As shown, each of the removers 402, 403, 404 includes one or more retention portions 422 that extend inwardly from the sidewall 418 into the passage 420. In some versions, the retention portions 422 can be formed integrally with the sidewall 418 and/or can be disposed symmetrically therearound. For example, the remover 402, 403, 404 can include any number of retention portions 422, such as 1, 2, 3, 4, 8, or more retention portions 422.

In a first form shown in Figs. 9 and 10, the remover 402 can be configured to grip and retain the non-rigid outer wall of the needle shield 266 within the cap 240. In this form, the retention portions 422 can be tabs that are integral with the sidewall 418 and angled into the passage 420. For example, the tabs 422 can be angled to extend into the passage 420 about a fourth of a diameter of the sidewall 418. In one example, the tabs 422 can connect to the sidewall 418 at an edge that extends along the circumference of the sidewall 418 generally perpendicular to the longitudinal axis thereof, so that the tabs 422 extend in a distal direction within the passage 420. As shown, the tabs 422 can have a curved configuration similar to the curvature of the sidewall 418. If desired, the tabs 422 can include pointed ends 424 to better grip the needle shield 266. The pointed ends 424 allow the remover 402 to indent or pierce the non-rigid outer wall of the needle shield 266 to thereby retain the needle shield 266 within the cap 240. In some versions, the tabs 422 can have a thin cross-section or edges of the tabs 422 can be sharpened to increase the gripping effectiveness of the remover 402. As shown, the tabs 422 are disposed in an intermediate position along an axial length of the remover 402 with the pointed ends 424 thereof disposed in a distal half of the remover 402. This positioning allows the end of the needle shield 266 to be inserted through a majority of the remover 402 before the pointed ends 424 of the tabs 422 indent and begin to grip the needle shield 266. Thereafter, the needle shield 266 can be forced to a desired end position within the cap 240 with the tabs 422 dragging along the non-rigid outer wall of the needle shield 266 before establishing a final position. Further, the tabs 422 can have a distal length greater than a third of an axial length of the remover 402. In the illustrated form, the remover 402 includes three tabs 422 arrayed symmetrically about the sidewall 418 in a single plane that is perpendicular to the longitudinal axis of the remover 402.

In a second form shown in Fig. 11, the remover 403 can be configured to grip and retain the non-rigid outer wall of the needle shield 266 within the cap 240. In this form, the retention portions 422 can be tabs that are integral with the sidewall 418 and extend from a distal edge 426 of the sidewall 418. As shown, the tabs 422 can be angled radially inwardly to extend in a distal direction into a path of an object inserted through the passage 420 of the sidewall 418. As shown, the tabs 422 can have a curved configuration similar to the curvature of the sidewall 418. If desired, the tabs 422 can include pointed ends 424 to better grip the needle shield 266. Similar to the above form, the pointed ends 424 allow the remover 402 to indent or pierce the non-rigid outer wall of the needle shield 266 to thereby retain the needle shield 266 within the cap 240. In alternative versions, the tabs 422 can have a flat distal edge. In some versions, the tabs 422 can have a thin cross-section or edges of the tabs 422 can be sharpened to increase the gripping effectiveness of the remover 402. The distal positioning of the tabs 422 allows the end of the needle shield 266 to be inserted through the remover 402 before the pointed ends 424 of the tabs 422 indent and begin to grip the needle shield 266. Thereafter, the needle shield 266 can be forced to a desired end position within the cap 240 with the tabs 422 dragging along the non-rigid outer wall of the needle shield 266 before establishing a final position. Further, the tabs 422 can have a distal length greater than a third of an axial length of the remover 403. In the illustrated form, the remover 402 includes three tabs 422 arrayed symmetrically about the sidewall 418 in a single plane that is perpendicular to the longitudinal axis L of the remover 402.

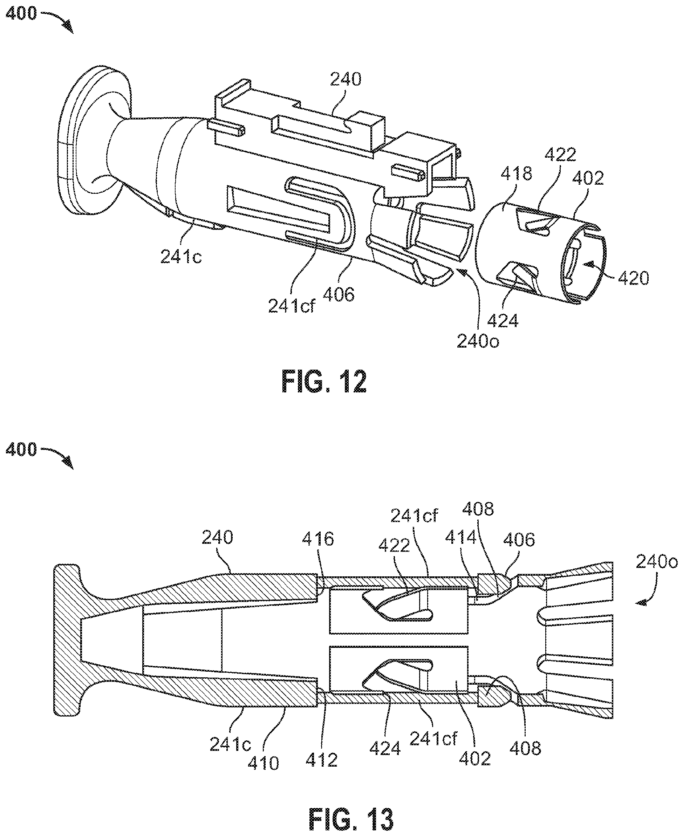

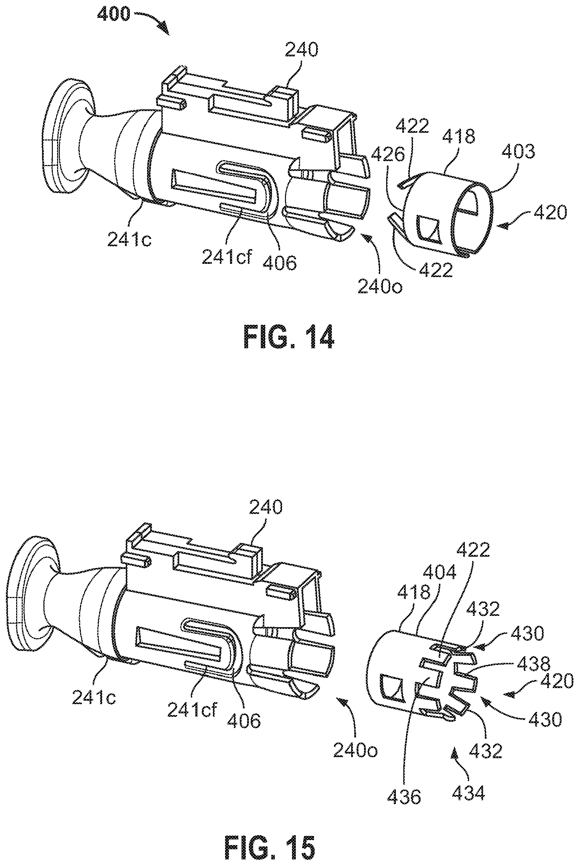

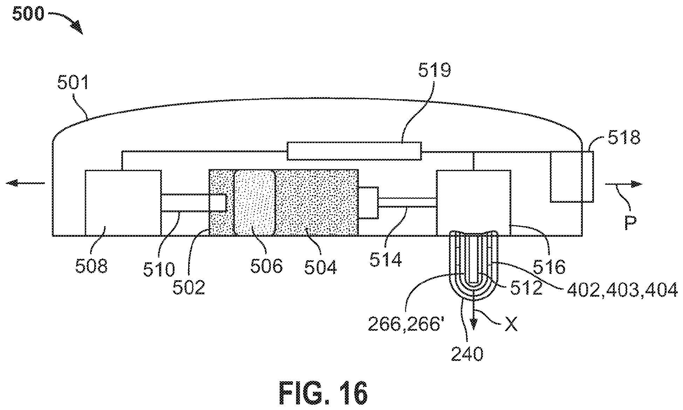

In a third form shown in Fig. 12, the remover 404 can be configured to grip and retain the rigid outer wall of the needle shield 266' within the cap 240. In this form, the retention portions 422 are tabs that extend into the passage 420 in a direction generally orthogonal to the longitudinal axis, and can be integral with the sidewall 418. The tabs 422 have a generally rectangular configuration, which can have interior edges 438 that run generally parallel with the longitudinal axis of the remover 403. In one example, the tabs 422 can be arrayed symmetrically about the sidewall 418 in a single plane that is perpendicular to the longitudinal axis L of the remover 404. The interior edges 438 of the tabs 422 form an interior diameter smaller than an outer diameter of the rigid outer wall of the needle shield 266', which can be slightly, e.g., between about 0.1-1.0 mm, smaller than an outer diameter of the rigid outer wall of the needle shield 266'. With this configuration, when the needle shield 266' is inserted through the remover 404, the edges 438 provide a compression force on the needle shield 266' that is distributed across the extended axial length of the interior edges 438. As shown, the remover 404 includes eight tabs 422 which further distributes the compressive force around a circumference of the needle shield 266'. This arrangement advantageously retains the rigid outer wall of the needle shield 266' without relying on points of force. In the illustrated form, the tabs 422 are provided on the remover 404 by a crenellation configuration 428 that has a repeating pattern of spaces 430 extending between upstanding portions 432 of the sidewall 418. As shown, the crenellation configuration 428 is formed at a proximal end 434 of the sidewall 418, so that the tabs 422 engage the needle shield 266' as it is inserted into the remover 404. By one approach, the tabs 422 can be formed by manipulating material from the spaces 430 of the crenellation configuration 428 to bend to a radially inwardly extending configuration. With this configuration, the tabs 422 extend from one of the longitudinal edges 436 of the upstanding portions 432. Although the crenellation pattern 428 is shown with a pattern of eight spaces 430 and protruding portions 432, more or fewer can be utilized as desired. Further, the tabs 422 can have an axial length greater than a fourth of an axial length of the remover 402.

With this configuration, a user can select a desired remover 402, 403, 404 that is configured for the particular needle shield 266, 266' intended for the autoinjector system 100 and insert the remover 402, 403, 404 in an orientation so that the retention portions 422 are configured to retain the needle shield 266, 266' within the cap 240 when the cap 240 is extracted from the cassette 200. Thereafter, the cap 240 can be coupled to the cassette 200 as described above so that the needle shield 266, 266' is inserted through the remover 402, 403, 404. As the needle shield 266, 266' is inserted through the remover 402, 403, 404, the retention portions 422 engage and grip the outer wall of the needle shield 266, 266', whether rigid or non-rigid. As such, when operation of the autoinjector system 100 is desired, a user can extract the cap 240 to thereby uncouple the needle shield 266, 266' from the syringe 260 to expose the injection needle 265.

For all of the above forms, the remover 402, 403, 404 and cap 240 can be made from different materials. For example, the remover 402, 403, 404 can be metal while the cap 240 can be plastic. Additionally, in some versions, multiple removers 402, 403, 404 can be provided in a kit so that a physician or other person assembling the cap assembly 400 can adapt the autoinjector system 100 for needle shields 266, 266' having rigid and non-rigid outer walls as desired.

Any of the above components can also be assembled in a particular method. For example, a cap assembly 400 including a cap housing 240 having an internal cavity 410 with a rearward opening 240o and a remover 402, 403, 404 having a sidewall 418 with a generally annular configuration can be provided. Thereafter, a physician or other person assembling the cap assembly 400 can orient the remover 402, 403, 404 to be coincident with a longitudinal axis of the cap housing 240 and insert the remover 402, 403, 404 into the internal cavity 410 of the cap housing 240 so that a plurality of retention portions 422 of the remover 402, 403, 404 extend radially inwardly from the sidewall 418 within the internal cavity 410. Inserting the remover 402, 403, 404 can include inserting the remover 402, 403, 404 between the arms 241cf of the cap housing 240 and the arms 241cf can be configured to flex outwardly and resiliently return inwardly to retain the remover 402, 403, 404 within the internal cavity 410. If desired, the method of assembly can include selecting one of the removers 402, 403, 404 based at least in part on an exterior wall of a needle shield 266, 266' intended for use with the cap assembly 400, where the removers 402, 403, 404 each having a different plurality of retention portions 422 as described above. In some versions, the method can include restricting linear and rotational motion of the needle shield 266, 266' with the remover 402, 403, 404, and/or providing a cassette 200, a syringe 260 that is disposed within the cassette 200 and includes a reservoir 262 containing a drug 267 and a needle 265 fluidly coupled to the reservoir 262, and a needle shield 266, 266' disposed over the needle 265 and coupling the cap assembly 400 to the cassette 200 so that the needle shield 266, 266' is disposed within the internal cavity 410 of the cap housing 240 and through the remover 402, 403, 404 with the plurality of retention portions 422 gripping an outer wall of the needle shield 266, 266'. Further, the method can include extracting the cap assembly 400 from the cassette 200 to thereby uncouple the needle shield 266, 266' from the syringe 260 and expose the needle 265.

Although the above disclosure has been described with reference to the structure and operation of autoinjector drug delivery devices, the disclosure is also suitable for and can be incorporated within on body drug delivery devices. As illustrated in Fig. 13, on body injectors 400 can have a horizontally oriented configuration with drug delivery components disposed generally along a horizontal plane P within a housing 401 of the devices 400. With these devices 400, the housing 401 has a low profile with a larger width than height so that when a user positions the housing 401 on the skin, the components are spread out over an area of the skin rather than stacked as with the above embodiments. The drug delivery components can include a reservoir 402 having a drug 404 contained therein, which can be removably disposed within the housing 401, a stopper 406 disposed within the reservoir 402 and sildably movable therein along the horizontal plane P, a drive mechanism 408 coupled to a plunger 410 to drive the stopper 406 through the reservoir 402, a needle 412 oriented along an axis X that extends generally perpendicular to the horizontal plane P, a flow path 414 fluidly coupling the reservoir 402 to the needle 412, and a needle insertion mechanism 416 configured to insert the needle 412 to a desired subcutaneous depth within the user. As commonly configured, one or more of the components of the device 400, such as the drive mechanism 408 and needle insertion mechanism 416, can be operable in response to actuation of a user input device 418 accessible on an exterior of the housing 401. Pursuant to this, the device 400 can include electronic components, such as a controller 419, to control operation of one or more of the drug delivery components. As described above, the device 400 of this form can also include a cap assembly 400 that includes a cap housing 240 and a remover 402, 403, 404. The device 400 can further include a needle shield 266, 266' disposed over the needle 412 in a storage state, where the needle shield 266, 266' is engaged and retained by the remover 402, 403, 404. The needle shield 266, 266' is removable by extraction of the cap assembly 400 from the device 400. Of course, it will be understood that some components can be disposed partially or entirely above or below the horizontal plane P extending generally centrally through the housing 401 and still be considered to have a horizontally oriented configuration. Suitable drive mechanisms include, but are not limited to, springs, gas sources, phase changing materials, motors, or other electromechanical systems. Example on body injector devices are described in US Serial No. 62/536,911, filed July 25, 2017.

The above description describes various components for drug delivery devices and methods for use and/or assembly associated with a drug delivery device. It should be clear that the drug delivery devices or methods can further comprise use of a medicament listed below with the caveat that the following list should neither be considered to be all inclusive nor limiting. The medicament will be contained in a reservoir. In some instances, the reservoir is a primary container that is either filled or pre-filled for treatment with the medicament. The primary container can be a cartridge or a pre-filled syringe.

For example, the drug delivery device or more specifically the reservoir of the device may be filled with colony stimulating factors, such as granulocyte colony-stimulating factor (G-CSF). Such G-CSF agents include, but are not limited to, Neupogen^{®} (filgrastim) and Neulasta^{®} (pegfilgrastim). In various other embodiments, the drug delivery device may be used with various pharmaceutical products, such as an erythropoiesis stimulating agent (ESA), which may be in a liquid or a lyophilized form. An ESA is any molecule that stimulates erythropoiesis, such as Epogen^{®} (epoetin alfa), Aranesp^{®} (darbepoetin alfa), Dynepo^{®} (epoetin delta), Mircera^{®} (methyoxy polyethylene glycol-epoetin beta), Hematide^{®}, MRK-2578, INS-22, Retacrit^{®} (epoetin zeta), Neorecormon^{®} (epoetin beta), Silapo^{®} (epoetin zeta), Binocrit^{®} (epoetin alfa), epoetin alfa Hexal, Abseamed^{®} (epoetin alfa), Ratioepo^{®} (epoetin theta), Eporatio^{®} (epoetin theta), Biopoin^{®} (epoetin theta), epoetin alfa, epoetin beta, epoetin zeta, epoetin theta, and epoetin delta, as well as the molecules or variants or analogs thereof as disclosed in the following patents or patent applications: U.S. Patent Nos. 4,703,008; 5,441,868; 5,547,933; 5,618,698; 5,621,080; 5,756,349; 5,767,078; 5,773,569; 5,955,422; 5,986,047; 6,583,272; 7,084,245; and 7,271,689; and PCT Publication Nos. WO 91/05867; WO 95/05465; WO 96/40772; WO 00/24893; WO 01/81405; and WO 2007/136752.

An ESA can be an erythropoiesis stimulating protein. As used herein, "erythropoiesis stimulating protein" means any protein that directly or indirectly causes activation of the erythropoietin receptor, for example, by binding to and causing dimerization of the receptor. Erythropoiesis stimulating proteins include erythropoietin and variants, analogs, or derivatives thereof that bind to and activate erythropoietin receptor; antibodies that bind to erythropoietin receptor and activate the receptor; or peptides that bind to and activate erythropoietin receptor. Erythropoiesis stimulating proteins include, but are not limited to, epoetin alfa, epoetin beta, epoetin delta, epoetin omega, epoetin iota, epoetin zeta, and analogs thereof, pegylated erythropoietin, carbamylated erythropoietin, mimetic peptides (including EMP1/hematide), and mimetic antibodies. Exemplary erythropoiesis stimulating proteins include erythropoietin, darbepoetin, erythropoietin agonist variants, and peptides or antibodies that bind and activate erythropoietin receptor (and include compounds reported in U.S. Publication Nos. 2003/0215444 and 2006/0040858) as well as erythropoietin molecules or variants or analogs thereof as disclosed in the following patents or patent applications: U.S. Patent Nos. 4,703,008; 5,441,868; 5,547,933; 5,618,698; 5,621,080; 5,756,349; 5,767,078; 5,773,569; 5,955,422; 5,830,851; 5,856,298; 5,986,047; 6,030,086; 6,310,078; 6,391,633; 6,583,272; 6,586,398; 6,900,292; 6,750,369; 7,030,226; 7,084,245; and 7,217,689; U.S. Publication Nos. 2002/0155998; 2003/0077753; 2003/0082749; 2003/0143202; 2004/0009902; 2004/0071694; 2004/0091961; 2004/0143857; 2004/0157293; 2004/0175379; 2004/0175824; 2004/0229318; 2004/0248815; 2004/0266690; 2005/0019914; 2005/0026834; 2005/0096461; 2005/0107297; 2005/0107591; 2005/0124045; 2005/0124564; 2005/0137329; 2005/0142642; 2005/0143292; 2005/0153879; 2005/0158822; 2005/0158832; 2005/0170457; 2005/0181359; 2005/0181482; 2005/0192211; 2005/0202538; 2005/0227289; 2005/0244409; 2006/0088906; and 2006/0111279; and PCT Publication Nos. WO 91/05867; WO 95/05465; WO 99/66054; WO 00/24893; WO 01/81405; WO 00/61637; WO 01/36489; WO 02/014356; WO 02/19963; WO 02/20034; WO 02/49673; WO 02/085940; WO 03/029291; WO 2003/055526; WO 2003/084477; WO 2003/094858; WO 2004/002417; WO 2004/002424; WO 2004/009627; WO 2004/024761; WO 2004/033651; WO 2004/035603; WO 2004/043382; WO 2004/101600; WO 2004/101606; WO 2004/101611; WO 2004/106373; WO 2004/018667; WO 2005/001025; WO 2005/001136; WO 2005/021579; WO 2005/025606; WO 2005/032460; WO 2005/051327; WO 2005/063808; WO 2005/063809; WO 2005/070451; WO 2005/081687; WO 2005/084711; WO 2005/103076; WO 2005/100403; WO 2005/092369; WO 2006/50959; WO 2006/02646; and WO 2006/29094.

Examples of other pharmaceutical products for use with the device may include, but are not limited to, antibodies such as Vectibix^{®} (panitumumab), Xgeva^{™} (denosumab) and Prolia^{™} (denosamab); other biological agents such as Enbrel^{®} (etanercept, TNF-receptor /Fc fusion protein, TNF blocker), Neulasta^{®} (pegfilgrastim, pegylated filgastrim, pegylated G-CSF, pegylated hu-Met-G-CSF), Neupogen^{®} (filgrastim , G-CSF, hu-MetG-CSF), and Nplate^{®} (romiplostim); small molecule drugs such as Sensipar^{®} (cinacalcet). The device may also be used with a therapeutic antibody, a polypeptide, a protein or other chemical, such as an iron, for example, ferumoxytol, iron dextrans, ferric glyconate, and iron sucrose. The pharmaceutical product may be in liquid form, or reconstituted from lyophilized form.

Among particular illustrative proteins are the specific proteins set forth below, including fusions, fragments, analogs, variants or derivatives thereof:
OPGL specific antibodies, peptibodies, and related proteins, and the like (also referred to as RANKL specific antibodies, peptibodies and the like), including fully humanized and human OPGL specific antibodies, particularly fully humanized monoclonal antibodies, including but not limited to the antibodies described in PCT Publication No. WO 03/002713, including OPGL specific antibodies and antibody related proteins, particularly those having the sequences set forth therein, particularly, but not limited to, those denoted therein: 9H7; 18B2; 2D8; 2E11; 16E1; and 22B3, including the OPGL specific antibodies having either the light chain of SEQ ID NO:2 as set forth therein in Figure 2 and/or the heavy chain of SEQ ID NO:4, as set forth therein in Figure 4, each of which is disclosed in the foregoing publication;
Myostatin binding proteins, peptibodies, and related proteins, and the like, including myostatin specific peptibodies, particularly those described in U.S. Publication No. 2004/0181033 and PCT Publication No. WO 2004/058988, particularly in parts pertinent to myostatin specific peptibodies, including but not limited to peptibodies of the mTN8-19 family, including those of SEQ ID NOS:305-351, including TN8-19-1 through TN8-19-40, TN8-19 con1 and TN8-19 con2; peptibodies of the mL2 family of SEQ ID NOS:357-383; the mL15 family of SEQ ID NOS:384-409; the mL17 family of SEQ ID NOS:410-438; the mL20 family of SEQ ID NOS:439-446; the mL21 family of SEQ ID NOS:447-452; the mL24 family of SEQ ID NOS:453-454; and those of SEQ ID NOS:615-631, each of which is disclosed in the foregoing publication;
IL-4 receptor specific antibodies, peptibodies, and related proteins, and the like, particularly those that inhibit activities mediated by binding of IL-4 and/or IL-13 to the receptor, including those described in PCT Publication No. WO 2005/047331 or PCT Application No. PCT/US2004/37242 and in U.S. Publication No. 2005/112694, particularly in parts pertinent to IL-4 receptor specific antibodies, particularly such antibodies as are described therein, particularly, and without limitation, those designated therein: L1H1; L1H2; L1H3; L1H4; L1H5; L1H6; L1H7; L1H8; L1H9; L1H10; L1H11; L2H1; L2H2; L2H3; L2H4; L2H5; L2H6; L2H7; L2H8; L2H9; L2H10; L2H11; L2H12; L2H13; L2H14; L3H1; L4H1; L5H1; L6H1, each of which is disclosed in the foregoing publication;
Interleukin 1-receptor 1 ("IL1-R1") specific antibodies, peptibodies, and related proteins, and the like, including but not limited to those described in U.S. Publication No. 2004/097712, in parts pertinent to IL1-R1 specific binding proteins, monoclonal antibodies in particular, especially, without limitation, those designated therein: 15CA, 26F5, 27F2, 24E12, and 10H7, each of which is disclosed in the aforementioned publication;
Ang2 specific antibodies, peptibodies, and related proteins, and the like, including but not limited to those described in PCT Publication No. WO 03/057134 and U.S. Publication No. 2003/0229023, particularly in parts pertinent to Ang2 specific antibodies and peptibodies and the like, especially those of sequences described therein and including but not limited to: L1(N); L1(N) WT; L1(N) 1K WT; 2xL1(N); 2xL1(N) WT; Con4 (N), Con4 (N) 1K WT, 2xCon4 (N) 1K; L1C; L1C 1K; 2xL1C; Con4C; Con4C 1K; 2xCon4C 1K; Con4-L1 (N); Con4-L1C; TN-12-9 (N); C17 (N); TN8-8(N); TN8-14 (N); Con 1 (N), also including anti-Ang 2 antibodies and formulations such as those described in PCT Publication No. WO 2003/030833, particularly Ab526; Ab528; Ab531; Ab533; Ab535; Ab536; Ab537; Ab540; Ab543; Ab544; Ab545; Ab546; A551; Ab553; Ab555; Ab558; Ab559; Ab565; AbF1AbFD; AbFE; AbFJ; AbFK; AbG1D4; AbGC1E8; AbH1C12; AblA1; AbIF; AblK, AblP; and AbIP, in their various permutations as described therein, each of which is disclosed in the foregoing publication;
NGF specific antibodies, peptibodies, and related proteins, and the like including, in particular, but not limited to those described in U.S. Publication No. 2005/0074821 and U.S. Patent No. 6,919,426, particularly as to NGF-specific antibodies and related proteins in this regard, including in particular, but not limited to, the NGF-specific antibodies therein designated 4D4, 4G6, 6H9, 7H2, 14D10 and 14D11, each of which is disclosed in the foregoing publication;
CD22 specific antibodies, peptibodies, and related proteins, and the like, such as those described in U.S. Patent No. 5,789,554, including CD22 specific antibodies and related proteins, particularly human CD22 specific antibodies, such as but not limited to humanized and fully human antibodies, including but not limited to humanized and fully human monoclonal antibodies, particularly including but not limited to human CD22 specific IgG antibodies, such as, for instance, a dimer of a human-mouse monoclonal hLL2 gamma-chain disulfide linked to a human-mouse monoclonal hLL2 kappa-chain, including, but limited to, for example, the human CD22 specific fully humanized antibody in Epratuzumab, CAS registry number 501423-23-0;
IGF-1 receptor specific antibodies, peptibodies, and related proteins, and the like, such as those described in PCT Publication No. WO 06/069202, including IGF-1 receptor specific antibodies and related proteins, including but not limited to the IGF-1 specific antibodies therein designated L1H1, L2H2, L3H3, L4H4, L5H5, L6H6, L7H7, L8H8, L9H9, L10H10, L11H11, L12H12, L13H13, L14H14, L15H15, L16H16, L17H17, L18H18, L19H19, L20H20, L21H21, L22H22, L23H23, L24H24, L25H25, L26H26, L27H27, L28H28, L29H29, L30H30, L31H31, L32H32, L33H33, L34H34, L35H35, L36H36, L37H37, L38H38, L39H39, L40H40, L41H41, L42H42, L43H43, L44H44, L45H45, L46H46, L47H47, L48H48, L49H49, L50H50, L51H51, L52H52, and IGF-1R-binding fragments and derivatives thereof, each of which is disclosed in the foregoing publication;
Also among non-limiting examples of anti-IGF-1R antibodies for use in the methods and compositions of the present invention are each and all of those described in:
   U.S. Publication No. 2006/0040358 (published February 23, 2006), 2005/0008642 (published January 13, 2005), 2004/0228859 (published November 18, 2004), including but not limited to, for instance, antibody 1A (DSMZ Deposit No. DSM ACC 2586), antibody 8 (DSMZ Deposit No. DSM ACC 2589), antibody 23 (DSMZ Deposit No. DSM ACC 2588) and antibody 18 as described therein;
   PCT Publication No. WO 06/138729 (published December 28, 2006) and WO 05/016970 (published February 24, 2005), and Lu et al. (2004), J. Biol. Chem. 279:2856-2865, including but not limited to antibodies 2F8, A12, and IMC-A12 as described therein;
   PCT Publication No. WO 07/012614 (published February 1, 2007), WO 07/000328 (published January 4, 2007), WO 06/013472 (published February 9, 2006), WO 05/058967 (published June 30, 2005), and WO 03/059951 (published July 24, 2003);
   U.S. Publication No. 2005/0084906 (published April 21, 2005), including but not limited to antibody 7C10, chimaeric antibody C7C10, antibody h7C10, antibody 7H2M, chimaeric antibody *7C10, antibody GM 607, humanized antibody 7C10 version 1, humanized antibody 7C10 version 2, humanized antibody 7C10 version 3, and antibody 7H2HM, as described therein;
   U.S. Publication Nos. 2005/0249728 (published November 10, 2005), 2005/0186203 (published August 25, 2005), 2004/0265307 (published December 30, 2004), and 2003/0235582 (published December 25, 2003) and Maloney et al. (2003), Cancer Res. 63:5073-5083, including but not limited to antibody EM164, resurfaced EM164, humanized EM164, huEM164 v1.0, huEM164 v1.1, huEM164 v1.2, and huEM164 v1.3 as described therein;
   U.S. Patent No. 7,037,498 (issued May 2, 2006), U.S. Publication Nos. 2005/0244408 (published November 30, 2005) and 2004/0086503 (published May 6, 2004), and Cohen, et al. (2005), Clinical Cancer Res. 11:2063-2073, e.g., antibody CP-751,871, including but not limited to each of the antibodies produced by the hybridomas having the ATCC accession numbers PTA-2792, PTA-2788, PTA-2790, PTA-2791, PTA-2789, PTA-2793, and antibodies 2.12.1, 2.13.2, 2.14.3, 3.1.1, 4.9.2, and 4.17.3, as described therein;
   U.S. Publication Nos. 2005/0136063 (published June 23, 2005) and 2004/0018191 (published January 29, 2004), including but not limited to antibody 19D12 and an antibody comprising a heavy chain encoded by a polynucleotide in plasmid 15H12/19D12 HCA (y4), deposited at the ATCC under number PTA-5214, and a light chain encoded by a polynucleotide in plasmid 15H12/19D12 LCF (κ), deposited at the ATCC under number PTA-5220, as described therein; and
   U.S. Publication No. 2004/0202655 (published October 14, 2004), including but not limited to antibodies PINT-6A1, PINT-7A2, PINT-7A4, PINT-7A5, PINT-7A6, PINT-8A1, PINT-9A2, PINT-11A1, PINT-11A2, PINT-11A3, PINT-11A4, PINT-11A5, PINT-11A7, PINT-11A12, PINT-12A1, PINT-12A2, PINT-12A3, PINT-12A4, and PINT-12A5, as described therein; particularly as to the aforementioned antibodies, peptibodies, and related proteins and the like that target IGF-1 receptors;
   B-7 related protein 1 specific antibodies, peptibodies, related proteins and the like ("B7RP-1," also is referred to in the literature as B7H2, ICOSL, B7h, and CD275), particularly B7RP-specific fully human monoclonal IgG2 antibodies, particularly fully human IgG2 monoclonal antibody that binds an epitope in the first immunoglobulin-like domain of B7RP-1, especially those that inhibit the interaction of B7RP-1 with its natural receptor, ICOS, on activated T cells in particular, especially, in all of the foregoing regards, those disclosed in U.S. Publication No. 2008/0166352 and PCT Publication No. WO 07/011941, including such antibodies and related proteins, including but not limited to antibodies designated therein as follow: 16H (having light chain variable and heavy chain variable sequences SEQ ID NO:1 and SEQ ID NO:7 respectively therein); 5D (having light chain variable and heavy chain variable sequences SEQ ID NO:2 and SEQ ID NO:9 respectively therein); 2H (having light chain variable and heavy chain variable sequences SEQ ID NO:3 and SEQ ID NO:10 respectively therein); 43H (having light chain variable and heavy chain variable sequences SEQ ID NO:6 and SEQ ID NO:14 respectively therein); 41H (having light chain variable and heavy chain variable sequences SEQ ID NO:5 and SEQ ID NO:13 respectively therein); and 15H (having light chain variable and heavy chain variable sequences SEQ ID NO:4 and SEQ ID NO:12 respectively therein), each of which is disclosed in the foregoing publication;
   IL-15 specific antibodies, peptibodies, and related proteins, and the like, such as, in particular, humanized monoclonal antibodies, particularly antibodies such as those disclosed in U.S. Publication Nos. 2003/0138421; 2003/023586; and 2004/0071702; and U.S. Patent No. 7,153,507, including IL-15 specific antibodies and related proteins, including peptibodies, including particularly, for instance, but not limited to, HuMax IL-15 antibodies and related proteins, such as, for instance, 146B7;
   IFN gamma specific antibodies, peptibodies, and related proteins and the like, especially human IFN gamma specific antibodies, particularly fully human anti-IFN gamma antibodies, such as, for instance, those described in U.S. Publication No. 2005/0004353, including IFN gamma specific antibodies, particularly, for example, the antibodies therein designated 1118; 1118*; 1119; 1121; and 1121*. The entire sequences of the heavy and light chains of each of these antibodies, as well as the sequences of their heavy and light chain variable regions and complementarity determining regions, are disclosed in the foregoing publication and in Thakur et al. (1999), Mol. Immunol. 36:1107-1115. In addition, description of the properties of these antibodies is provided in the foregoing publication. Specific antibodies include those having the heavy chain of SEQ ID NO:17 and the light chain of SEQ ID NO:18; those having the heavy chain variable region of SEQ ID NO:6 and the light chain variable region of SEQ ID NO:8; those having the heavy chain of SEQ ID NO:19 and the light chain of SEQ ID NO:20; those having the heavy chain variable region of SEQ ID NO:10 and the light chain variable region of SEQ ID NO:12; those having the heavy chain of SEQ ID NO:32 and the light chain of SEQ ID NO:20; those having the heavy chain variable region of SEQ ID NO:30 and the light chain variable region of SEQ ID NO:12; those having the heavy chain sequence of SEQ ID NO:21 and the light chain sequence of SEQ ID NO:22; those having the heavy chain variable region of SEQ ID NO:14 and the light chain variable region of SEQ ID NO:16; those having the heavy chain of SEQ ID NO:21 and the light chain of SEQ ID NO:33; and those having the heavy chain variable region of SEQ ID NO:14 and the light chain variable region of SEQ ID NO:31, as disclosed in the foregoing publication. A specific antibody contemplated is antibody 1119 as disclosed in the foregoing U.S. publication and having a complete heavy chain of SEQ ID NO:17 as disclosed therein and having a complete light chain of SEQ ID NO:18 as disclosed therein;
   TALL-1 specific antibodies, peptibodies, and the related proteins, and the like, and other TALL specific binding proteins, such as those described in U.S. Publication Nos. 2003/0195156 and 2006/0135431, including TALL-1 binding proteins, particularly the molecules of Tables 4 and 5B, each of which is disclosed in the foregoing publications;
   Parathyroid hormone ("PTH") specific antibodies, peptibodies, and related proteins, and the like, such as those described in U.S. Patent No. 6,756,480, particularly in parts pertinent to proteins that bind PTH;
   Thrombopoietin receptor ("TPO-R") specific antibodies, peptibodies, and related proteins, and the like, such as those described in U.S. Patent No. 6,835,809, particularly in parts pertinent to proteins that bind TPO-R;
   Hepatocyte growth factor ("HGF") specific antibodies, peptibodies, and related proteins, and the like, including those that target the HGF/SF:cMet axis (HGF/SF:c-Met), such as the fully human monoclonal antibodies that neutralize hepatocyte growth factor/scatter (HGF/SF) described in U.S. Publication No. 2005/0118643 and PCT Publication No. WO 2005/017107, huL2G7 described in U.S. Patent No. 7,220,410 and OA-5d5 described in U.S. Patent Nos. 5,686,292 and 6,468,529 and in PCT Publication No. WO 96/38557, particularly in parts pertinent to proteins that bind HGF;
   TRAIL-R2 specific antibodies, peptibodies, related proteins and the like, such as those described in U.S. Patent No. 7,521,048, particularly in parts pertinent to proteins that bind TRAIL-R2;
   Activin A specific antibodies, peptibodies, related proteins, and the like, including but not limited to those described in U.S. Publication No. 2009/0234106, particularly in parts pertinent to proteins that bind Activin A;
   TGF-beta specific antibodies, peptibodies, related proteins, and the like, including but not limited to those described in U.S. Patent No. 6,803,453 and U.S. Publication No. 2007/0110747, particularly in parts pertinent to proteins that bind TGF-beta;
   Amyloid-beta protein specific antibodies, peptibodies, related proteins, and the like, including but not limited to those described in PCT Publication No. WO 2006/081171, particularly in parts pertinent to proteins that bind amyloid-beta proteins. One antibody contemplated is an antibody having a heavy chain variable region comprising SEQ ID NO:8 and a light chain variable region having SEQ ID NO:6 as disclosed in the foregoing publication;
   c-Kit specific antibodies, peptibodies, related proteins, and the like, including but not limited to those described in U.S. Publication No. 2007/0253951, particularly in parts pertinent to proteins that bind c-Kit and/or other stem cell factor receptors;
   OX40L specific antibodies, peptibodies, related proteins, and the like, including but not limited to those described in U.S. Publication No. 2006/0002929, particularly in parts pertinent to proteins that bind OX40L and/or other ligands of the OX40 receptor; and
   Other exemplary proteins, including Activase^{®} (alteplase, tPA); Aranesp^{®} (darbepoetin alfa); Epogen^{®} (epoetin alfa, or erythropoietin); GLP-1, Avonex^{®} (interferon beta-1a); Bexxar^{®} (tositumomab, anti-CD22 monoclonal antibody); Betaseron^{®} (interferon-beta); Campath^{®} (alemtuzumab, anti-CD52 monoclonal antibody); Dynepo^{®} (epoetin delta); Velcade^{®} (bortezomib); MLN0002 (anti- α4ß7 mAb); MLN1202 (anti-CCR2 chemokine receptor mAb); Enbrel^{®} (etanercept, TNF-receptor /Fc fusion protein, TNF blocker); Eprex^{®} (epoetin alfa); Erbitux^{®} (cetuximab, anti-EGFR / HER1 / c-ErbB-1); Genotropin^{®} (somatropin, Human Growth Hormone); Herceptin^{®} (trastuzumab, anti-HER2/neu (erbB2) receptor mAb); Humatrope^{®} (somatropin, Human Growth Hormone); Humira^{®} (adalimumab); insulin in solution; Infergen^{®} (interferon alfacon-1); Natrecor^{®} (nesiritide; recombinant human B-type natriuretic peptide (hBNP); Kineret^{®} (anakinra); Leukine^{®} (sargamostim, rhuGM-CSF); LymphoCide^{®} (epratuzumab, anti-CD22 mAb); Benlysta^{™} (lymphostat B, belimumab, anti-BlyS mAb); Metalyse^{®} (tenecteplase, t-PA analog); Mircera^{®} (methoxy polyethylene glycol-epoetin beta); Mylotarg^{®} (gemtuzumab ozogamicin); Raptiva^{®} (efalizumab); Cimzia^{®} (certolizumab pegol, CDP 870); Soliris^{™} (eculizumab); pexelizumab (anti-C5 complement); Numax^{®} (MEDI-524); Lucentis^{®} (ranibizumab); Panorex^{®} (17-1A, edrecolomab); Trabio^{®} (lerdelimumab); TheraCim hR3 (nimotuzumab); Omnitarg (pertuzumab, 2C4); Osidem^{®} (IDM-1); OvaRex^{®} (B43.13); Nuvion^{®} (visilizumab); cantuzumab mertansine (huC242-DM1); NeoRecormon^{®} (epoetin beta); Neumega^{®} (oprelvekin, human interleukin-11); Neulasta^{®} (pegylated filgastrim, pegylated G-CSF, pegylated hu-Met-G-CSF); Neupogen^{®} (filgrastim , G-CSF, hu-MetG-CSF); Orthoclone OKT3^{®} (muromonab-CD3, anti-CD3 monoclonal antibody); Procrit^{®} (epoetin alfa); Remicade^{®} (infliximab, anti-TNFα monoclonal antibody); Reopro^{®} (abciximab, anti-GP IIb/IIia receptor monoclonal antibody); Actemra^{®} (anti-IL6 Receptor mAb); Avastin^{®} (bevacizumab), HuMax-CD4 (zanolimumab); Rituxan^{®} (rituximab, anti-CD20 mAb); Tarceva^{®} (erlotinib); Roferon-A^{®}-(interferon alfa-2a); Simulect^{®} (basiliximab); Prexige^{®} (lumiracoxib); Synagis^{®} (palivizumab); 146B7-CHO (anti-IL15 antibody, see U.S. Patent No. 7,153,507); Tysabri^{®} (natalizumab, anti-α4integrin mAb); Valortim^{®} (MDX-1303, anti-B. anthracis protective antigen mAb); ABthrax^{™}; Vectibix^{®} (panitumumab); Xolair^{®} (omalizumab); ETI211 (anti-MRSA mAb); IL-1 trap (the Fc portion of human IgG1 and the extracellular domains of both IL-1 receptor components (the Type I receptor and receptor accessory protein)); VEGF trap (Ig domains of VEGFR1 fused to IgG1 Fc); Zenapax^{®} (daclizumab); Zenapax^{®} (daclizumab, anti-IL-2Rα mAb); Zevalin^{®} (ibritumomab tiuxetan); Zetia^{®} (ezetimibe); Orencia^{®} (atacicept, TACI-Ig); anti-CD80 monoclonal antibody (galiximab); anti-CD23 mAb (lumiliximab); BR2-Fc (huBR3 / huFc fusion protein, soluble BAFF antagonist); CNTO 148 (golimumab, anti-TNFα mAb); HGS-ETR1 (mapatumumab; human anti-TRAIL Receptor-1 mAb); HuMax-CD20 (ocrelizumab, anti-CD20 human mAb); HuMax-EGFR (zalutumumab); M200 (volociximab, anti-α5β1 integrin mAb); MDX-010 (ipilimumab, anti-CTLA-4 mAb and VEGFR-1 (IMC-18F1); anti-BR3 mAb; anti-C. difficile Toxin A and Toxin B C mAbs MDX-066 (CDA-1) and MDX-1388); anti-CD22 dsFv-PE38 conjugates (CAT-3888 and CAT-8015); anti-CD25 mAb (HuMax-TAC); anti-CD3 mAb (NI-0401); adecatumumab; anti-CD30 mAb (MDX-060); MDX-1333 (anti-IFNAR); anti-CD38 mAb (HuMax CD38); anti-CD40L mAb; anti-Cripto mAb; anti-CTGF Idiopathic Pulmonary Fibrosis Phase I Fibrogen (FG-3019); anti-CTLA4 mAb; anti-eotaxin1 mAb (CAT-213); anti-FGF8 mAb; anti-ganglioside GD2 mAb; anti-ganglioside GM2 mAb; anti-GDF-8 human mAb (MYO-029); anti-GM-CSF Receptor mAb (CAM-3001); anti-HepC mAb (HuMax HepC); anti-IFNα mAb (MEDI-545, MDX-1103); anti-IGF1R mAb; anti-IGF-1R mAb (HuMax-Inflam); anti-IL12 mAb (ABT-874); anti-IL12/IL23 mAb (CNTO 1275); anti-IL13 mAb (CAT-354); anti-IL2Ra mAb (HuMax-TAC); anti-IL5 Receptor mAb; anti-integrin receptors mAb (MDX-018, CNTO 95); anti-IP10 Ulcerative Colitis mAb (MDX-1100); anti-LLY antibody; BMS-66513; anti-Mannose Receptor/hCGβ mAb (MDX-1307); anti-mesothelin dsFv-PE38 conjugate (CAT-5001); anti-PD1mAb (MDX-1106 (ONO-4538)); anti-PDGFRα antibody (IMC-3G3); anti-TGFß mAb (GC-1008); anti-TRAIL Receptor-2 human mAb (HGS-ETR2); anti-TWEAK mAb; anti-VEGFR/Flt-1 mAb; anti-ZP3 mAb (HuMax-ZP3); NVS Antibody #1; and NVS Antibody #2.

Also included can be a sclerostin antibody, such as but not limited to romosozumab, blosozumab, or BPS 804 (Novartis). Further included can be therapeutics such as rilotumumab, bixalomer, trebananib, ganitumab, conatumumab, motesanib diphosphate, brodalumab, vidupiprant, panitumumab, denosumab, NPLATE, PROLIA, VECTIBIX or XGEVA. Additionally, included in the device can be a monoclonal antibody (IgG) that binds human Proprotein Convertase Subtilisin/Kexin Type 9 (PCSK9). Such PCSK9 specific antibodies include, but are not limited to, Repatha^{®} (evolocumab) and Praluent^{®} (alirocumab), as well as molecules, variants, analogs or derivatives thereof as disclosed in the following patents or patent applications: U.S. Patent No. 8,030,547, U.S. Publication No. 2013/0064825, WO2008/057457, WO2008/057458, WO2008/057459, WO2008/063382, WO2008/133647, WO2009/100297, WO2009/100318, WO2011/037791, WO2011/053759, WO2011/053783, WO2008/125623, WO2011/072263, WO2009/055783, WO2012/0544438, WO2010/029513, WO2011/111007, WO2010/077854, WO2012/088313, WO2012/101251, WO2012/101252, WO2012/101253, WO2012/109530, and WO2001/031007.

Also included can be talimogene laherparepvec or another oncolytic HSV for the treatment of melanoma or other cancers. Examples of oncolytic HSV include, but are not limited to talimogene laherparepvec (U.S. Patent Nos. 7,223,593 and 7,537,924); OncoVEXGALV/CD (U.S. Pat. No. 7,981,669); OrienX010 (Lei et al. (2013), World J. Gastroenterol., 19:5138-5143); G207, 1716; NV1020; NV12023; NV1034 and NV1042 (Vargehes et al. (2002), Cancer Gene Ther., 9(12):967-978).

Also included are TIMPs. TIMPs are endogenous tissue inhibitors of metalloproteinases (TIMPs) and are important in many natural processes. TIMP-3 is expressed by various cells or and is present in the extracellular matrix; it inhibits all the major cartilage-degrading metalloproteases, and may play a role in role in many degradative diseases of connective tissue, including rheumatoid arthritis and osteoarthritis, as well as in cancer and cardiovascular conditions. The amino acid sequence of TIMP-3, and the nucleic acid sequence of a DNA that encodes TIMP-3, are disclosed in U.S. Patent No. 6,562,596, issued May 13, 2003. Description of TIMP mutations can be found in U.S. Publication No. 2014/0274874 and PCT Publication No. WO 2014/152012.

Also included are antagonistic antibodies for human calcitonin gene-related peptide (CGRP) receptor and bispecific antibody molecule that target the CGRP receptor and other headache targets. Further information concerning these molecules can be found in PCT Application No. WO 2010/075238.

Additionally, bispecific T cell engager (BiTE^{®}) antibodies, e.g. BLINCYTO^{®} (blinatumomab), can be used in the device. Alternatively, included can be an APJ large molecule agonist e.g., apelin or analogues thereof in the device. Information relating to such molecules can be found in PCT Publication No. WO 2014/099984.

In certain embodiments, the medicament comprises a therapeutically effective amount of an anti-thymic stromal lymphopoietin (TSLP) or TSLP receptor antibody. Examples of anti-TSLP antibodies that may be used in such embodiments include, but are not limited to, those described in U.S. Patent Nos. 7,982,016, and 8,232,372, and U.S. Publication No. 2009/0186022. Examples of anti-TSLP receptor antibodies include, but are not limited to, those described in U.S. Patent No. 8,101,182. In particularly preferred embodiments, the medicament comprises a therapeutically effective amount of the anti-TSLP antibody designated as A5 within U.S. Patent No. 7,982,016.

Although the drug delivery devices, methods, and elements thereof, have been described in terms of exemplary embodiments, they are not limited thereto. The detailed description is to be construed as exemplary only and does not describe every possible embodiment of the invention because describing every possible embodiment would be impractical, if not impossible. Numerous alternative embodiments could be implemented, using either current technology or technology developed after the filing date of this patent that would still fall within the scope of the claims defining the invention. For example, components described herein with reference to certain kinds of drug delivery devices, such as on-body injector drug delivery devices or other kinds of drug delivery devices, can also be utilized in other kinds of drug delivery devices, such as autoinjector drug delivery devices.

Those skilled in the art will recognize that a wide variety of modifications, alterations, and combinations can be made with respect to the above described embodiments without departing from the scope of the invention, and that such modifications, alterations, and combinations are to be viewed as being within the ambit of the inventive concept defined by the claims.

## Claims

1. A drug delivery device (10) including a housing (11), an injection assembly, a cassette (200), a syringe (260) disposed within the cassette and comprising a reservoir (262) containing a drug and a needle (265) fluidly coupled to the reservoir, and a cap assembly kit (400);
the cap assembly kit comprising:
a first remover (402, 403) having a sidewall (418) defining a central passage and configured to receive a first type of needle shield (266), the first type of needle shield having a non-rigid outer wall, wherein the first remover includes a first plurality of retention portions extending inwardly into the central passage of the first remover, wherein the first plurality of retention portions comprises tabs (422) which are angled into the central passage and have distally oriented pointed ends configured to indent or pierce a portion of the first type of needle shield;
a second remover (404) having a sidewall defining a central passage and configured to receive a second type of needle shield (266'), the second type of needle shield having a rigid outer wall, wherein the second remover includes a second plurality of retention portions extending inwardly into the central passage of the second remover, wherein the second plurality of retention portions comprises tabs (422) which extend into the central passage in a direction generally orthogonal to a longitudinal axis of the remover, the tabs having a generally rectangular configuration and forming an interior diameter that is smaller than an outer diameter of a rigid outer wall of the second type of needle shield, such that the tabs are configured to provide a compression force on the second type of needle shield that is distributed across an axial length; and
**characterised in that** the cap assembly kit comprises a cap housing (240) having an internal cavity and a plurality of arms (241cf) extending along the longitudina axis thereof, the arms each being resiliently flexible and having an inwardly extending retention surface to engage the remover as the remover is inserted into the internal cavity of the cap housing past the retention surface, wherein the internal cavity is configured to interchangeably receive one of the first remover (402, 403) and the second remover (404).

2. The drug delivery device of claim 1, wherein the first remover and the second remover are each configured to be insertably mounted within the internal cavity of the cap housing.

3. The drug delivery device of claim 1or 2, wherein the first plurality of retention portions extend from a distal edge of the sidewall.

4. The drug delivery device of any one of claims 1 to 3, wherein the second plurality of retention portions comprise engagement edges that extend generally parallel to a longitudinal axis of the second remover.

5. The drug delivery device of claim 4, wherein the engagement edges have a blunted configuration.

6. The drug delivery device of any one of claims 1 to 5, wherein the second plurality of retention portions are disposed in a proximal axial position of the second remover.

7. The drug delivery device of any one of claims 1 to 6, wherein the sidewall comprises a crenellation configuration (428) with the plurality of retention portions extending radially inward from longitudinal edges of portions of the crenellation configuration.

8. A method of assembly for a drug delivery device cassette, the method comprising:
providing a cap assembly including a cap housing (240) having an internal cavity with a rearward opening, the cap housing further comprising a plurality of arms (241cf) extending along the longitudinal axis thereof, the arms each being resiliently flexible and having an inwardly extending retention surface to engage a remover as the remover is inserted into the internal cavity of the cap housing past the retention surface;
selecting one of a first remover (402, 403) configured to receive a first type of needle shield (266), the first type of needle shield having a non-rigid outer wall, wherein the first remover includes a first plurality of retention portions extending inwardly into the central passage of the first remover, wherein the first plurality of retention portions comprises tabs (422) which are angled into the central passage and have distally oriented pointed ends configured to indent or pierce a portion of the first type of needle shield; and a second remover (404) configured to receive a second type of needle shield (266'), the second type of needle shield having a rigid outer wall, wherein the second remover includes a second plurality of retention portions extending inwardly into the central passage of the second remover, wherein the second plurality of retention portions comprises tabs (422) which extend into the central passage in a direction generally orthogonal to a longitudinal axis of the remover, the tabs having a generally rectangular configuration and forming an interior diameter that is smaller than an outer diameter of a rigid outer wall of the second type of needle shield, such that the tabs are configured to provide a compression force on the second type of needle shield that is distributed across an axial length;
orienting the selected one of the first remover and second remover to be coincident with a longitudinal axis of the cap housing; and
inserting the selected one of the first remover and second remover into the internal cavity of the cap housing.

9. The method of claim 8, wherein the first and second removers each have a different plurality of retention portions (422) that extend radially inwardly from an annular sidewall thereof, and selecting the one of the first remover and the second remover comprises selecting the one of the first remover and the second remover based on whether the plurality of retention portions thereof are configured to grip a needle shield having a rigid or a non-rigid outer wall.

10. The method of claim 9, further comprising:
providing a cassette (200), a syringe (260) disposed within the cassette and comprising a reservoir (262) containing a drug and a needle (265) fluidly coupled to the reservoir, and a needle shield disposed over the needle; and
coupling the cap assembly to the cassette so that the needle shield is disposed within the internal cavity of the cap housing and through the selected one of the first remover and second remover with the plurality of retention portions gripping an outer wall of the needle shield.

11. The method of claim 10, further comprising restricting linear and rotational motion of the needle shield with the remover.

12. The method of claim 10 or 11, further comprising extracting the cap assembly from the cassette to thereby uncouple the needle shield from the syringe and the needle.

## Patentansprüche

1. Arzneimittelabgabevorrichtung (10) mit einem Gehäuse (11), einer Injektionsanordnung, einer Kassette (200), einer Spritze (260), die innerhalb der Kassette angeordnet ist und ein Reservoir (262) umfasst, das ein Arzneimittel und eine mit dem Reservoir fluidisch verbundene Nadel (265) enthält, und einem Kappenanordnungskit (400);
wobei das Kappenanordnungskit Folgendes umfasst:
einen ersten Entnehmer (402, 403) mit einer Seitenwand (418), die einen zentralen Durchgang definiert und so konfiguriert ist, dass sie eine erste Art von Nadelschutz (266) aufnimmt, wobei die erste Art von Nadelschutz eine nicht starre Außenwand aufweist, wobei der erste Entnehmer eine erste Vielzahl von Halteabschnitten umfasst, die sich nach innen in den zentralen Durchgang des ersten Entnehmers erstrecken, wobei die erste Vielzahl von Halteabschnitten Laschen (422) umfasst, die in den zentralen Durchgang hinein abgewinkelt sind und distal ausgerichtete, spitze Enden aufweisen, die so konfiguriert sind, dass sie einen Abschnitt der ersten Art von Nadelschutz eindrücken oder durchstechen;
einen zweiten Entnehmer (404) mit einer Seitenwand, die einen zentralen Durchgang definiert und so konfiguriert ist, dass sie eine zweite Art von Nadelschutz (266') aufnimmt, wobei die zweite Art von Nadelschutz eine starre Außenwand aufweist, wobei der zweite Entnehmer eine zweite Vielzahl von Halteabschnitten umfasst, die sich nach innen in den zentralen Durchgang des zweiten Entnehmers erstrecken, wobei die zweite Vielzahl von Halteabschnitten Laschen (422) umfasst, die sich in eine Richtung, die im Wesentlichen orthogonal zu einer Längsachse des Entnehmers verläuft, in den zentralen Durchgang erstrecken, wobei die Laschen eine im Wesentlichen rechteckige Konfiguration aufweisen und einen Innendurchmesser bilden, der kleiner ist als ein Außendurchmesser einer starren Außenwand der zweiten Art von Nadelschutz, so dass die Laschen so konfiguriert sind, dass sie eine Druckkraft auf die zweite Art von Nadelschutz ausüben, die über eine axiale Länge verteilt ist;
**dadurch gekennzeichnet, dass** das Kappenanordnungskit Folgendes umfasst
ein Kappengehäuse (240) mit einem inneren Hohlraum und einer Vielzahl von Armen (241cf), die sich entlang der Längsachse davon erstrecken, wobei die Arme jeweils elastisch biegsam sind und eine sich nach innen erstreckende Haltefläche aufweisen, um mit dem Entnehmer in Eingriff zu kommen, wenn der Entnehmer in den inneren Hohlraum des Kappengehäuses über die Haltefläche hinweg eingeführt wird, wobei der innere Hohlraum so konfiguriert ist, dass er abwechselnd entweder den ersten Entnehmer (402, 403) oder den zweiten Entnehmer (404) aufnehmen kann.

2. Arzneimittelabgabevorrichtung nach Anspruch 1, wobei der erste Entnehmer und der zweite Entnehmer jeweils so konfiguriert sind, dass sie einfügbar in dem inneren Hohlraum des Kappengehäuses angebracht werden können.

3. Arzneimittelabgabevorrichtung nach Anspruch 1 oder 2, wobei sich die erste Vielzahl von Halteabschnitten von einer distalen Kante der Seitenwand erstreckt.

4. Arzneimittelabgabevorrichtung nach einem der Ansprüche 1 bis 3, wobei die zweite Vielzahl von Halteabschnitten Eingriffskanten umfasst, die sich im Wesentlichen parallel zu einer Längsachse des zweiten Entnehmers erstrecken.

5. Arzneimittelabgabevorrichtung nach Anspruch 4, wobei die Eingriffskanten eine abgestumpfte Konfiguration aufweisen.

6. Arzneimittelabgabevorrichtung nach einem der Ansprüche 1 bis 5, wobei die zweite Vielzahl von Halteabschnitten in einer proximalen axialen Position des zweiten Entnehmers angeordnet ist.

7. Arzneimittelabgabevorrichtung nach einem der Ansprüche 1 bis 6, wobei die Seitenwand eine Zinnenkonfiguration (428) aufweist, wobei sich die Vielzahl von Halteabschnitten radial nach innen von Längskanten von Abschnitten der Zinnenkonfiguration erstreckt.

8. Verfahren zum Zusammenbau einer Kassette für eine Arzneimittelabgabevorrichtung, wobei das Verfahren Folgendes umfasst:
Bereitstellen einer Kappenanordnung, die ein Kappengehäuse (240) mit einem inneren Hohlraum mit einer hinteren Öffnung umfasst, wobei das Kappengehäuse ferner eine Vielzahl von Armen (241cf) umfasst, die sich entlang der Längsachse davon erstrecken, wobei die Arme jeweils elastisch biegsam sind und eine sich nach innen erstreckende Haltefläche aufweisen, um mit einem Entnehmer in Eingriff zu kommen, wenn der Entnehmer in den inneren Hohlraum des Kappengehäuses über die Haltefläche hinweg eingeführt wird;
Auswählen von einem eines ersten Entnehmers (402, 403), der zur Aufnahme einer ersten Art von Nadelschutz (266) konfiguriert ist, wobei die erste Art von Nadelschutz eine nicht starre Außenwand aufweist, wobei der erste Entnehmer eine erste Vielzahl von Halteabschnitten umfasst, die sich nach innen in den zentralen Durchgang des ersten Entnehmers erstrecken, wobei die erste Vielzahl von Halteabschnitten Laschen (422) umfasst, die in den zentralen Durchgang hinein abgewinkelt sind und distal ausgerichtete, spitze Enden aufweisen, die so konfiguriert sind, dass sie einen Abschnitt der ersten Art von Nadelschutz eindrücken oder durchstechen; und eines zweiten Entnehmers (404), der zur Aufnahme einer zweiten Art von Nadelschutz (266') konfiguriert ist, wobei die zweite Art von Nadelschutz eine starre Außenwand aufweist, wobei der zweite Entnehmer eine zweite Vielzahl von Halteabschnitten umfasst, die sich nach innen in den zentralen Durchgang des zweiten Entnehmers erstrecken, wobei die zweite Vielzahl von Halteabschnitten Laschen (422) umfasst, die sich in eine Richtung, die im Wesentlichen orthogonal zu einer Längsachse des Entnehmers verläuft, in den zentralen Durchgang erstrecken, wobei die Laschen eine im Wesentlichen rechteckige Konfiguration aufweisen und einen Innendurchmesser bilden, der kleiner ist als ein Außendurchmesser einer starren Außenwand der zweiten Art von Nadelschutz, so dass die Laschen so konfiguriert sind, dass sie eine Druckkraft auf die zweite Art von Nadelschutz ausüben, die über eine axiale Länge verteilt ist;
Ausrichten des ausgewählten ersten Entnehmers oder zweiten Entnehmers, so dass er mit einer Längsachse des Kappengehäuses übereinstimmt; und
Einsetzen des ausgewählten ersten Entnehmers oder zweiten Entnehmers in den inneren Hohlraum des Kappengehäuses.

9. Verfahren nach Anspruch 8, wobei der erste und der zweite Entnehmer jeweils eine unterschiedliche Vielzahl von Halteabschnitten (422) aufweisen, die sich von einer ringförmigen Seitenwand davon radial nach innen erstrecken, und wobei das Auswählen des ersten Entnehmers oder des zweiten Entnehmers das Auswählen des ersten Entnehmers oder des zweiten Entnehmers auf der Grundlage dessen umfasst, ob die Vielzahl von Halteabschnitten davon konfiguriert sind, um einen Nadelschutz mit einer starren oder einer nicht starren Außenwand zu greifen.

10. Verfahren nach Anspruch 9, ferner umfassend:
Bereitstellen einer Kassette (200), einer Spritze (260), die innerhalb der Kassette angeordnet ist und ein Reservoir (262) umfasst, das ein Arzneimittel und eine mit dem Reservoir fluidisch verbundene Nadel (265) enthält, und eines über der Nadel angeordneten Nadelschutzes; und
Koppeln der Kappenanordnung mit der Kassette, so dass der Nadelschutz innerhalb des inneren Hohlraums des Kappengehäuses und durch den ausgewählten ersten Entnehmer oder zweiten Entnehmer angeordnet ist, wobei die Vielzahl von Halteabschnitten eine Außenwand des Nadelschutzes greifen.

11. Verfahren nach Anspruch 10, ferner umfassend das Einschränken der linearen und rotatorischen Bewegung des Nadelschutzes mit dem Entnehmer.

12. Verfahren nach Anspruch 10 oder 11, ferner umfassend das Entfernen der Kappenanordnung aus der Kassette, um dadurch den Nadelschutz von der Spritze und der Nadel zu entkoppeln.

## Revendications

1. Dispositif d'administration de médicament (10) comprenant un boîtier (11), un ensemble d'injection, une cassette (200), une seringue (260) disposée à l'intérieur de la cassette et comprenant un réservoir (262) contenant un médicament et une aiguille (265) couplée de manière fluidique au réservoir, ainsi qu'un kit d'ensemble capuchon (400),
le kit d'ensemble capuchon comprenant :
un premier organe de retrait (402, 403) présentant une paroi latérale (418) définissant un passage central et conçu pour recevoir un premier type de protège-aiguille (266), le premier type de protège-aiguille présentant une paroi extérieure non rigide, le premier organe de retrait comprenant une première pluralité de portions de retenue s'étendant vers l'intérieur dans le passage central du premier organe de retrait, la première pluralité de portions de retenue comprenant des languettes (422) qui sont inclinées dans le passage central et présentent des extrémités pointues orientées distalement, conçues pour enfoncer ou percer une portion du premier type de protège-aiguille,
un deuxième organe de retrait (404) présentant une paroi latérale définissant un passage central et conçu pour recevoir un deuxième type de protège-aiguille (266'), le deuxième type de protège-aiguille présentant une paroi extérieure rigide, le deuxième organe de retrait comprenant une deuxième pluralité de portions de retenue s'étendant vers l'intérieur dans le passage central du deuxième organe de retrait, la deuxième pluralité de portions de retenue comprenant des languettes (422) qui s'étendent dans le passage central dans une direction globalement orthogonale à un axe longitudinal de l'organe de retrait, les languettes présentant une configuration globalement rectangulaire et formant un diamètre intérieur inférieur au diamètre extérieur d'une paroi extérieure rigide du deuxième type de protège-aiguille, de telle façon que les languettes sont conçues pour exercer une force de compression sur le deuxième type de protège-aiguille, laquelle est répartie sur une longueur axiale ;
**caractérisé en ce que** le kit d'ensemble capuchon comprend un boîtier de capuchon (240) présentant une cavité intérieure et une pluralité de bras (241cf) s'étendant le long de son axe longitudinal, les bras étant chacun élastiquement flexibles et présentant une surface de retenue s'étendant vers l'intérieur pour s'accoupler à l'organe de retrait tandis que l'organe de retrait est inséré dans la cavité intérieure du boîtier de capuchon au-delà de la surface de retenue, la cavité intérieure étant conçue pour recevoir de manière interchangeable soit le premier organe de retrait (402, 403), soit le deuxième organe de retrait (404).

2. Dispositif d'administration de médicament selon la revendication 1, dans lequel le premier organe de retrait et le deuxième organe de retrait sont chacun conçus pour être montés de manière amovible à l'intérieur de la cavité intérieure du boîtier de capuchon.

3. Dispositif d'administration de médicament selon la revendication 1 ou 2, dans lequel la première pluralité de portions de retenue s'étend à partir d'un bord distal de la paroi latérale.

4. Dispositif d'administration de médicament selon l'une quelconque des revendications 1 à 3, dans lequel la deuxième pluralité de portions de retenue comprend des bords d'accouplement qui s'étendent de manière globalement parallèle à un axe longitudinal du deuxième organe de retrait.

5. Dispositif d'administration de médicament selon la revendication 4, dans lequel les bords d'accouplement présentent une configuration émoussée.

6. Dispositif d'administration de médicament selon l'une quelconque des revendications 1 à 5, dans lequel la deuxième pluralité de portions de retenue est disposée en une position axiale proximale par rapport au deuxième organe de retrait.

7. Dispositif d'administration de médicament selon l'une quelconque des revendications 1 à 6, dans lequel la paroi latérale présente une configuration crénelée (428), la pluralité de portions de retenue s'étendant radialement vers l'intérieur à partir des bords longitudinaux de portions de la configuration crénelée.

8. Procédé d'assemblage d'une cassette pour dispositif d'administration de médicament, le procédé comprenant :
la fourniture d'un ensemble capuchon comprenant un boîtier de capuchon (240) présentant une cavité intérieure dotée d'une ouverture vers l'arrière, le boîtier de capuchon comprenant en outre une pluralité de bras (241cf) s'étendant le long de son axe longitudinal, les bras étant chacun élastiquement flexibles et présentant une surface de retenue s'étendant vers l'intérieur pour s'accoupler à un organe de retrait tandis que l'organe de retrait est inséré dans la cavité intérieure du boîtier de capuchon au-delà de la surface de retenue,
la sélection : d'un premier organe de retrait (402, 403) conçu pour recevoir un premier type de protège-aiguille (266), le premier type de protège-aiguille présentant une paroi extérieure non rigide, le premier organe de retrait comprenant une première pluralité de portions de retenue s'étendant vers l'intérieur dans le passage central du premier organe de retrait, la première pluralité de portions de retenue comprenant des languettes (422) qui sont inclinées dans le passage central et présentent des extrémités pointues orientées distalement, conçues pour enfoncer ou percer une portion du premier type de protège-aiguille ; ou d'un deuxième organe de retrait (404) conçu pour recevoir un deuxième type de protège-aiguille (266'), le deuxième type de protège-aiguille présentant une paroi extérieure rigide, le deuxième organe de retrait comprenant une deuxième pluralité de portions de retenue s'étendant vers l'intérieur dans le passage central du deuxième organe de retrait, la deuxième pluralité de portions de retenue comprenant des languettes (422) qui s'étendent dans le passage central dans une direction globalement orthogonale à un axe longitudinal de l'organe de retrait, les languettes présentant une configuration globalement rectangulaire et formant un diamètre intérieur inférieur au diamètre extérieur d'une paroi extérieure rigide du deuxième type de protège-aiguille, de telle façon que les languettes sont conçues pour exercer une force de compression sur le deuxième type de protège-aiguille, laquelle est répartie sur une longueur axiale ;
l'orientation de l'organe de retrait sélectionné, parmi le premier et le deuxième organe de retrait, de telle façon qu'il coïncide avec l'axe longitudinal du boîtier de capuchon, et
l'insertion de l'organe de retrait sélectionné, parmi le premier et le deuxième organe de retrait, dans la cavité intérieure du boîtier de capuchon.

9. Procédé selon la revendication 8, dans lequel le premier et le deuxième organe de retrait présentent chacun une pluralité différente de portions de retenue (422) s'étendant radialement vers l'intérieur à partir d'une paroi latérale annulaire de ceux-ci, et la sélection du premier ou du deuxième organe de retrait comprend la sélection du premier ou du deuxième organe de retrait en fonction du fait que sa pluralité de portions de retenue est conçue pour saisir un protège-aiguille présentant une paroi extérieure rigide ou non rigide.

10. Procédé selon la revendication 9, comprenant en outre :
la fourniture d'une cassette (200), d'une seringue (260) disposée à l'intérieur de la cassette et comprenant un réservoir (262) contenant un médicament et une aiguille (265) couplée de manière fluidique au réservoir, ainsi qu'un protège-aiguille disposé sur l'aiguille, et
le couplage de l'ensemble capuchon à la cassette de telle façon que le protège-aiguille est disposé à l'intérieur de la cavité intérieure du boîtier de capuchon et à travers le premier organe de retrait ou le deuxième organe de retrait sélectionné, la pluralité de portions de retenue saisissant une paroi extérieure du protège-aiguille.

11. Procédé selon la revendication 10, comprenant en outre une limitation du déplacement linéaire et en rotation du protège-aiguille grâce à l'organe de retrait.

12. Procédé selon la revendication 10 ou 11, comprenant en outre l'extraction de l'ensemble capuchon de la cassette pour ainsi désaccoupler le protège-aiguille de la seringue et de l'aiguille.
